# EUROPÄISCHE PATENTSCHRIFT

(11) **EP 0 563 010 B1**
(45) Veröffentlichungstag und Bekanntmachung des Hinweises auf die Patenterteilung: **28.04.1999**
(21) Anmeldenummer: 93810184.7
(22) Anmeldetag: 15.03.1993
(51) Int. Cl.: C07H 21/00, C07H 19/04

(54) **Verfahren zur Herstellung von Nukleosiden**
Method of preparation of nucleosides
Méthode de la préparation des nucléosides

(30) Priorität: 23.03.1992 CH 906/92
(43) Veröffentlichungstag der Anmeldung: 29.09.1993
(73) Patentinhaber: Novartis AG, 4058 Basel (CH); Novartis-Erfindungen Verwaltungsgesellschaft m.b.H., 1235 Wien (AT)
(72) Erfinder: Brill, Wolfgang K. D., Dr., W-7800 Freiburg-Waltershofen (DE)

(56) Entgegenhaltungen:
- SYNTHESIS, 1991, STUTTGART DE Seiten 201 - 204 M.FRONEMAN ET. AL. 'New synthesis of phosphorous and phosphoric acid esters'
- JOURNAL OF THE AMERICAN CHEMICAL SOCIETY, Bd.110, 1988, WASHINGTON, DC US Seiten 181 - 185 F.H.WESTHEIMER ET. AL. 'Rates and mechanisms of hydrolysis of esters of phosphorous acid'
- SCIENCE, Bd.230, Nr.4723, 18. Oktober 1985, LANCASTER, PA US Seiten 281 - 285 M.H.CARUTHERS 'Gene synthesis machines: DNA chemistry and its uses'
- CENTRAL PATENTS INDEX, BASIC ABSTRACTS JOURNAL Section Ch, Week 7446, Derwent Publications Ltd., London, GB; Class B07, AN 80093V & JP-A-52 029 749 (ASAHI CHEMICAL IND CO) 3. August 1977

## Beschreibung

Die vorliegende Erfindung betrifft ein Verfahren zur Herstellung von natürlichen oder synthetischen Nukleosiden, Nukleosidanalogen oder Oligonukleotiden mit mindestens zwei gleichen oder verschiedenen solcher Nukleosidmonomeren.

Heute werden viele Oligonukleotide definierter Sequenz nach dem Phosphittriester-Verfahren synthetisiert. Hierbei wird ein Oligonukleotid von seinem 3'-Terminus aus sukzessive in 5'-Richtung synthetisiert. Die Synthese beginnt mit der Anbindung des 3'-terminalen Nukleosids, welches eine Schutzgruppe an seiner 5'-Hydroxylgruppe und, wenn nötig, an seiner Nukleosidbase trägt, an eine chemisch inerte feste Phase. Anschliessend wird die 5'-Hydroxyschutzgruppe abgespalten. Durch Umsatz mit einem 3'-Phosphoramidit eines adäquat geschützten Nukleosids erfolgt die Bildung der internukleotidischen Bindung. Der resultierende Phosphittriester wird anschliessend zu der gewünschten Internukleotidbindung oxidiert. Nun kann der nächste Synthesekreislauf mit der Abspaltung der 5'-terminalen Schutzgruppe des auf der Festphase immobilisierten Dinukleotids eingeleitet werden. Die Synthesekreisläufe werden so lange wiederholt, bis man zu dem fertigen, an der Festphase gebundenen Oligonukleotid gelangt. Die Synthese endet mit der Abspaltung der Schutzgruppen an den Nukleosidbasen, Hydroxygruppen und den internukleotidischen Bindungen (Caruthers, M.H., Science 230;281-285, 1985). Je nach Art des Phosphoramidits enthält das eingesetzte Nukleosid oder Oligonukleotid zum Beispiel die Gruppen der Formeln

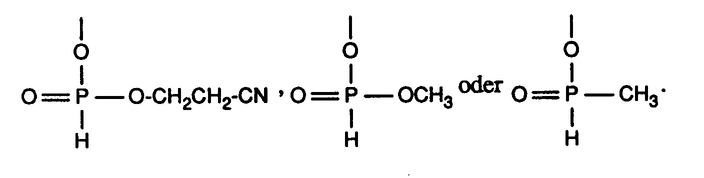

Problematisch bei diesem Syntheseverfahren ist die Kondensationsreaktion zwischen dem Phosphoramidit und der freien Hydroxygruppe der auf der Festphase immobilisierten Nukleoside oder Oligonukleotide. Bei diesem Reaktionsschritt muss auf peinlichsten Ausschluss von Wasser geachtet werden. Selbst durch die Verwendung von absolutierten Lösungsmitteln ist die Wasserfreiheit nicht vollständig gewährleistet. Deshalb kommt es bei dem Kopplungsschritt der Festphasenoligonukleotidsynthese zur Überführung erheblicher Mengen an Nukleosidphosphoramidit in Nukleosidphosphoramidit-Hydrolysat, zum Beispiel das Nukleosid-cyanoethylhydrogenphosphonat. Letzteres nimmt an der Kopplungsreaktion nicht teil. Um dennoch hohe Kopplungsausbeuten zu erreichen, müssen die Phosphoramidite in hohen Überschüssen eingesetzt werden. Bei modernen DNA-Syntheseautomaten werden bis zu 2- bis 20-fache Überschüsse an Nukleosid-phosphoramidit relativ zu der Menge der an der Festphase immobilisierten Hydroxykomponente eingesetzt. Hieraus ergibt sich eine Ausbeute an Internukleotidbindung von maximal 20 % relativ zum eingesetzten Phosphoramidit. Minimal 80 % der eingesetzten Nukleoside werden als Nukleosid-phosphoramidit-Hydrolysat verworfen. Dieses Problem kann auch durch apparitive Verbesserungen wie zum Beispiel speziellen Dosiervorrichtungen nicht vollständig beseitigt werden, siehe Chemische Rundschau Nr. 37, Seite 11 (1992).

Böhringer (Böhringer, M.P., Dissertation, Eidgenössische Technische Hochschule, ETH Nr. 9377, 1991) lässt das Hydrolysat eines Methylphosphoramidits eines Nukleosidanalogons mit Natronlauge zum Nukleosidanalogon mit einer freien Hydroxygruppe reagieren. Unter den von ihm verwendeten Hydrolysebedingungen werden jedoch die Schutzgruppen der Nukleosidbasen abgespalten.

Auch die von Predvoditelev et al. (Predvoditelev, D.A., Tyuganova, M.A., Nifant'ev, E.E., Rogovin, Z.A., Zh. Prikl. Khim. 40:171-177, 1967), Nifnt'ev et al. (Nifnt'ev, E.E., Predvoditelev, D.A., Fursenko, I.V., Zh. Obsh. Khim. 51:2435-2441, 1981), Troev und Simeonov (Troev, K., Simeonov, M., Phosphorus and Sulfur 19:363-367, 1984) und Westheimer et al. (Westheimer, F.H., Huang, S., Covitz, F., J. Am. Chem. Soc. 110:181-185, 1988) beschriebenen Verfahren zur Umesterung von nicht nukleotidischen Dialkylphosphiten haben alle den Nachteil, dass bei Anwendung auf geschützte Nukleoside als Nebenreaktion die Schutzgruppen der Nukleinsäurebasen leicht abgespalten werden. So wird in diesen Verfahren eine erhöhte Temperatur benötigt, was der generellen Anwendung wegen thermischer Zersetzungsreaktionen, beispielsweise die thermische Abspaltung von Schutzgruppen, insbesondere die Detritylierung, im Wege steht.

Ein weiteres Verfahren ist die Lewis-Säure-katalysierte Umesterung mit Terakis-benzyloxy-titan, welches bei 40°C innerhalb 72 Stunden mit mässigen Ausbeuten durchgeführt wird und bei die Hydrolyseempfindlichkeit des Titanats besonders nachteilig ist (Froneman, M., Modro, T.A., Synthesis 201-204, 1991).

Überraschenderweise wird nun durch die vorliegende Erfindung ein Verfahren zur Verfügung gestellt, Nukleosid-hydrogenphosphonate oder -hydrogenphosphinate in Nukleoside zu überführen, welche eine freie Hydroxylgruppe an jener Position haben, an der sich die Hydrogenphosphonat- bzw. -phosphinatfunktion befunden hatte. Bei dem erfindungsgemässen Verfahren wird die Hydrogenphosphonatgruppe bzw. Hydrogenphosphinatgruppe hochselektiv gegenüber anderen Acylschutzgruppen auf einen Alkohol übertragen. Die Selektivität der Abspaltung kann in manchen Fällen so gross sein, dass selbst nach mehrtägiger Einwirkung unter den gegebenen Reaktionsbedingungen die Schutzgruppen der Nukleosidbasen nicht abgespalten werden. Bei dieser Reaktion werden darüberhinaus hohe Ausbeuten erzielt. Das erfindungsgemässe Verfahren ermöglicht erstmals die Wiederverwertung der bei der Nukleotidkopplung nach dem gebräuchlichen Phosphittriesterverfahren anfallenden Nukleosidüberschüsse während des routinemässigen Dauerbetriebs der manuellen oder automatisierten Oligonukleotidsynthese.

Die vorliegende Erfindung betrifft insbesondere ein Verfahren zur Herstellung von natürlichen oder synthetischen Nukleosiden, Nukleosidanalogen oder Oligonukleotiden aus mindestens zwei solcher Nukleoside und/oder Nukleosidanalogen, deren Grundgerüst einen gegebenenfalls substituierten Rest einer Nukleinbase B und eine geschützte Hydroxylgruppe der Formel R₁-O-, worin R₁ für eine Schutzgruppe steht, enthält, oder die mit dem Sauerstoffatom einer Hydroxylgruppe direkt oder über eine Brückengruppe an ein festes Trägermaterial gebunden sind, aus Nukleosidmonomeren oder aus Oligonukleotiden, an deren Grundgerüst neben besagten Resten eine Gruppe der Formeln I, Ia oder Ib 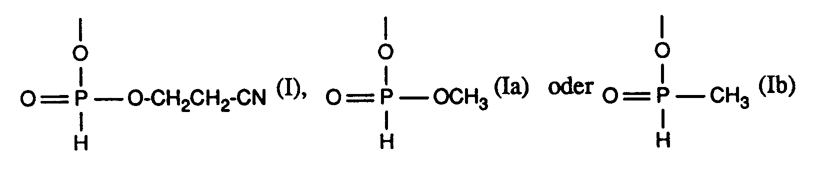 gebunden ist, durch Überführung der Gruppe der Formeln I, Ia oder Ib in eine Hydroxylgruppe -OH, indem man die Nukleoside oder Oligonukleotide, gegebenenfalls in einem inerten organischen Lösungsmittel, mit einem Überschuss eines aliphatischen, cycloaliphatischen, araliphatischen oder aromatischen Alkohols mit 1 bis 30 C-Atomen, einem Polyol mit 2 bis 50 C-Atomen oder einem polymeren Polyol in Gegenwart einer mindestens katalytischen Menge einer anorganischen Base oder einer organischen Stickstoffbase umsetzt, wobei die Base einen pK-Wert von 4 bis 10 aufweist.

Die Gruppen der Formeln I, Ia und Ib können in den 3'- oder 5'-Stellungen der Nukleoside bzw. Oligonukleotide gebunden sein, wobei die Gruppe R₁O- oder das feste Trägermaterial in den 5'- bzw. 3'-Positionen gebunden ist. Vorzugsweise ist die Gruppe der Formel I in der 5'-Position gebunden, die Gruppe der Formel Ib in der 3'-Stellung gebunden und die Gruppe der Formel Ia in den 3'- oder 5'-Positionen gebunden.

Für das erfindungsgemässe Verfahren geeignete Nukleoside, Nukleosidanaloge oder Oligonukleotide mit einer der Knüpfung der Nukleotidbindung dienenden vorzugsweise sekundären OH-Gruppe sind in grosser Vielzahl bekannt und in der Fachliteratur beschrieben, beispielsweise in Townsend (Townsend, L.B., Hrsg., Chemistry of Nucleosides and Nucleotides 1, Plenum Press, New York, 1988) oder nach bekannten Verfahren herstellbar. Es kann sich bei ihnen im allgemeinen um ein gegebenenfalls mit -O- oder - S- unterbrochenes offenkettiges C-Gerüst oder carbacyclisches oder O- bzw. S-heterocyclisches Grundgerüst mit einer Nukleinbase B handeln.

Das offenkettige Kohlenstoff-Gerüst kann zum Beispiel 3 bis 12, bevorzugt 3 bis 6 Kohlenstoff-Atome enthalten. Bei den carbacyclischen und heterocyclischen Grundgerüsten kann es sich zum Beispiel um monocyclische Ringsysteme mit 3 bis 12, bevorzugt 3 bis 8 und besonders bevorzugt 4 oder 5 Ringkohlenstoffatomen handeln. Es kann sich auch um bicyclische bis tetracyclische Systeme mit zum Beispiel 5 bis 16, bevorzugt 8 bis 16 Kohlenstoff-Atomen handeln. Die Grundgerüste können weitere Substituenten enthalten, zum Beispiel geschützte OH-Gruppen.

Wenn die Nukleinbase B einen Purinrest oder ein Analoges davon darstellt, so kann es sich um Reste der Formeln II, IIa, IIb, IIc, IId oder IIe handeln, 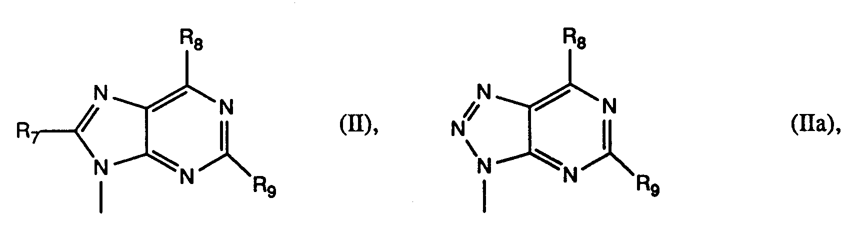 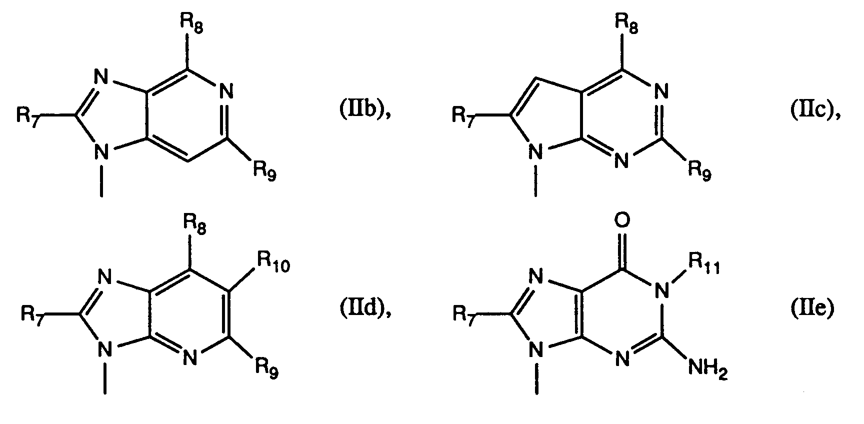 worin R₇ für H, Cl, Br, NH₂ oder OH steht, und R₈, R₉ und R₁₀ unabhängig voneinander H, OH, SH, NH₂, NHNH₂, NHOH, NHOAlkyl mit 1 bis 12 C-Atomen, F, Cl, Br, Alkyl oder Hydroxyalkyl oder Aminoalkyl oder Alkoxy oder Alkylthio mit 1 bis 12 C-Atomen, wobei die Hydroxyl- und Aminogruppen unsubstituiert oder mit einer Schutzgruppe substituiert sind, Phenyl, Benzyl, Primäramino mit 1 bis 20 C-Atomen oder Sekundäramino mit 2 bis 30 C-Atomen bedeuten, und R₁₁ H oder C₁-C₄-Alkyl darstellt.

Das Primäramino enthält bevorzugt 1 bis 12 und besonders bevorzugt 1 bis 6 C-Atome, und das Sekundäramino bevorzugt 2 bis 12 und besonders bevorzugt 2 bis 6 C-Atome.

Einige Beispiele für Alkyl, Alkoxy, Alkylthio, Hydroxyalkyl und Aminoalkyl, die bevorzugt 1 bis 6 C-Atome enthalten, sind Methyl, Ethyl und die Isomeren von Propyl, Butyl, Pentyl, Hexyl, Heptyl, Octyl, Nonyl, Decyl, Undecyl und Dodecyl, sowie entsprechende Alkoxy-, Alkylthio-, Hydroxyalkyl- und Aminoalkylreste. Das Alkyl, Alkoxy, Alkylthio, Hydroxyalkyl und Aminoalkyl enthält besonders bevorzugt 1 bis 4 C-Atome. Bevorzugte Alkyl-, Alkoxy-, Alkylthio-, Hydroxyalkyl- und Aminoalkylreste sind Methyl, Ethyl, n- und i-Propyl, n-, i- und t-Butyl, Methoxy, Ethoxy, Methylthio und Ethylthio, Aminomethyl, Aminoethyl, Hydroxymethyl und Hydroxyethyl.

Bei dem Primäramino und Sekundäramino kann es sich zum Beispiel um Reste der Formel R₁₄R₁₅N handeln, worin R₁₄ für H steht oder unabhängig die Bedeutung von R₁₅ hat, und R₁₅ C₁-C₂₀-, bevorzugt C₁-C₁₂- und besonders bevorzugt C₁-C₆-Alkyl, -Aminoalkyl, -Hydroxyalkyl; Carboxyalkyl oder Carbalkoxyalkyl, wobei die Carbalkoxygruppe 2 bis 8 C-Atome enthält und die Alkylgruppe 1 bis 6, bevorzugt 1 bis 4 C-Atome; C₂-C₂₀-, bevorzugt C₂-C₁₂- und besonders bevorzugt C₂-C₆-Alkenyl; Phenyl, Mono- oder Di-(C₁ -C₄-Alkyl-oder -Alkoxy)phenyl, Benzyl, Mono- oder Di-(C₁-C₄-Alkyl- oder -Alkoxy)benzyl; oder 1,2-, 1,3- oder 1,4-Imidazolyl-C₁-C₆-Alkyl darstellt, oder R₁₄ und R₁₅ zusammen Tetra-oder Pentamethylen, 3-oxa- 1,5-pentylen, -CH₂-NR₁₆-CH₂CH₂- oder -CH₂CH₂-NR₁₆-CH₂CH₂- darstellen, worin R₁₆ für H oder C₁-C₄-Alkyl steht. Die Aminogruppe im Aminoalkyl kann mit ein oder zwei C₁-C₄-Alkyl oder -Hydroxyalkylgruppen substituiert sein. Die Hydroxylgruppe im Hydroxyalkyl kann mit C₁-C₄-Alkyl verethert sein.

Beispiele für Alkyl sind zuvor angegeben worden. Beispiele für Aminoalkyl sind Aminomethyl, Aminoethyl, 1-Aminoprop-2-yl oder -3-yl, 1-Amino-but-2-yl oder -3-yl oder -4-yl, N-Methyl- oder N,N-Dimethyl- oder N-Ethyl- oder N,N-Diethyl- oder N-2-Hydroxyethyl-oder N,N-Di-2-hydroxyethylaminomethyl oder -aminoethyl oder -aminopropyl oder -aminobutyl. Beispiele für Hydroxyalkyl sind Hydroxymethyl, 1 -Hydroxy-eth-2-yl, 1-Hydroxy-prop-2- oder -3-yl, 1-Hydroxy-but-2-yl, -3-yl oder -4-yl. Beispiele für Carboxyalkyl sind Carboxymethyl, Carboxyethyl, Carboxypropyl und Carboxybutyl, und Beispiele für Carbalkoxyalkyl sind diese mit Methyl oder Ethyl veresterten Carboxyalkylgruppen. Beispiele für Alkenyl sind Allyl, But-1-en-3-yl oder -4-yl, Pent-3- oder 4-en-1-yl oder -2-yl, Hex-3- oder -4- oder -5-en-1-yl oder -2-yl. Beispiele für Alkyl-und Alkoxyphenyl beziehungsweise -benzyl sind Methylphenyl, Dimethylphenyl, Ethylphenyl, Diethylphenyl, Methylbenzyl, Dimethylbenzyl, Ethylbenzyl, Diethylbenzyl, Methoxyphenyl, Dimethoxyphenyl, Ethoxyphenyl, Diethoxyphenyl, Methoxybenzyl, Dimethoxybenzyl, Ethoxybenzyl, Diethoxybenzyl. Beispiele für Imidazolylalkyl, in dem die Alkylgruppe bevorzugt 2 bis 4 C-Atome enthält, sind 1,2-, 1,3- oder 1,4-Imidazolylethyl oder - n-propyl oder -n-butyl. R₁₆ stellt bevorzugt H, Methyl oder Ethyl dar.

Bevorzugte Beispiele für Primäramino und Sekundäramino sind Methyl-, Ethyl-, Dimethyl-, Diethyl-, Allyl-, Mono- oder Di-(1-hydroxy-eth-2-yl)-, Phenyl- und Benzylamino, Acetylamino und Benzoylamino.

In einer bevorzugten Ausführungsform stellt R₇ Wasserstoff dar. In einer anderen bevorzugten Ausführungsform stellt R₁₀ Wasserstoff dar. In einer weiteren bevorzugten Ausführungsform bedeuten R₈ und R₉ unabhängig voneinander H, F, Cl, Br, OH, SH, NH₂, NHOH, NHNH₂, Methylamino, Dimethylamino, Benzoylamino, Methoxy, Ethoxy und Methylthio.

Einige Beispiele für Analoge der Purinreihe sind neben Purin Adenin; N-Methyladenin, N-Benzyladenin, 2-Methyladenin, 2-Methylthioadenin, 2-Aminoadenin, 3-Carbaadenin, 7-Carbaadenin, 1-Carbaadenin, 6-Hydroxypurin, 2-Amino-6-chlorpurin, 2-Amino-6-methylthiopurin, 2-Amino-6-hydroxypurin, 3-Carba-6-chlorpurin, Guanin, 2-Methylguanin. Besonders bevorzugt sind Adenin, 2-Aminoadenin und Guanin.

Wenn die Nukleinbase B einen analogen Pyrimidinrest darstellt, so handelt es sich bevorzugt um Uracil-, Thymin- und Cytosinreste der Formeln III, IIIa und IIIb, 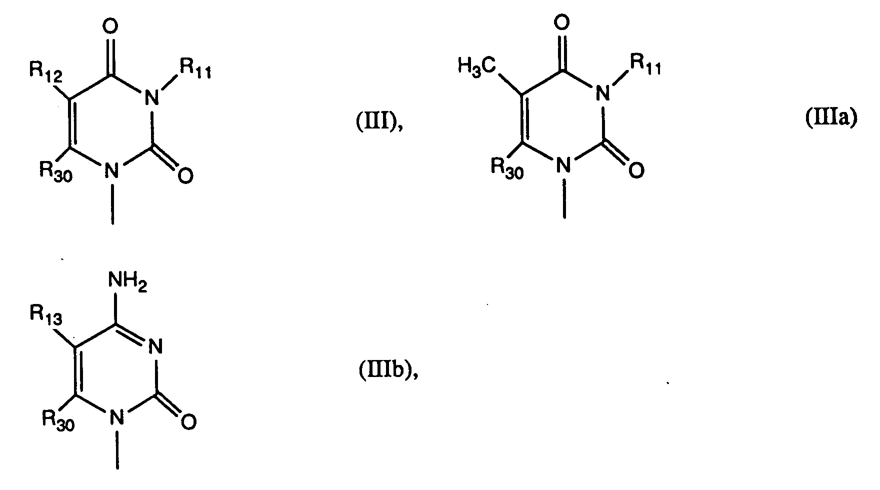 worin R₁₁ H oder C₁-C₄-Alkyl bedeutet, und R₁₂, R₁₃ und R₃₀ unabhängig voneinander die zuvor für R₈ angegebene Bedeutung haben, einschliesslich der Bevorzugungen, und die Wasserstoffatome der NH₂-Gruppe in Formel IIIb mit C₁-C₆-Alkyl oder Benzoyl substituiert sein können, sowie die Dihydroderivate der Reste der Formeln III, IIIa und IIIb. Bevorzugt stellt R₁₂ H, C₁-C₆-Alkyl oder Hydroxyalkyl, F, Cl, Br, NH₂, Benzoylamino, Mono- oder Di-C₁-C₆-alkylamino dar und R₁₃ stellt bevorzugt H, C₁-C₆-Alkyl oder -Alkoxy oder -Hydroxyalkyl, F, Cl, Br, NH₂, Benzoylamino, Mono- oder Di-C₁-C₆-alkylamino dar.

R₁₁ steht bevorzugt für H oder Methyl. R₁₂ bedeutet bevorzugt H, F, Cl, Br, NH₂, NHCH₃, N(CH₃)₂ oder C₁-C₄-Alkyl. R₁₃ stellt bevorzugt H, C₁-C₄-Alkyl, besonders Methyl, oder NH₂, NHCH₃ oder (CH₃)₂N dar.

Einige Beispiele für Pyrimidinanaloge sind Uracil, Thymin, Cytosin, 5-Fluoruracil, 5-Chloruracil, 5-Bromuracil, Dihydrouracil, Pseudouracil, 1-Methylpseudouracil, 5-Methyluracil, 3-Methylcytosin und 5-Methylcytosin.

Im Rahmen der vorliegenden Erfindung sind mit Schutzgruppen allgemein in der Fachwelt bekannte Schutzgruppen gemeint. Beispiele für solche Schutzgruppen sind; C₁-C₈-Alkyl ; mono- oder bicyclisches C₇-C₁₂-Aralkyl; mono- oder bicyclisches C₇-C₁₂-Aralkoxy; mono- oder bicyclisches C₇-C₁₂-Haloaralkyl; Diphenylmethyl; Diphenylmethyl, substituiert mit 1 bis 4 Methyl oder Methoxy; Triphenylmethyl; Triphenylmethyl, substituiert mit 1 bis 6 Methyl oder Methoxy, oder 1 bis 3 tert.-Butyl; Xanthenyl, substituiert mit Phenyl oder Naphthyl; -Si(R₄)(R₅)(R₆), worin (R₄)(R₅) und (R₆) unabhängig voneinander C₁-C₂₀ -Alkyl, Benzyl oder Phenyl bedeuten; R-C(O)-, worin R C₁-C₆-Alkyl, Benzyl, Benzyl, substituiert mit Methyl, Methoxy oder Halogen, C₁-C₁₂-Alkoxy, C₁-C₁₂-Alkoxy, substituiert mit Fluoren, Phenyloxy, Phenyloxy, substituiert mit Methyl, Methoxy oder Halogen, Benzyloxy oder Benzyloxy, substituiert mit Methyl, Methoxy oder Halogen bedeutet; R₁₇-SO₂-, worin R₁₇ C₁-C₁₂-Alkyl, C₅-C₆-Cycloalkyl, Phenyl, Phenyl, substituiert mit C₁-C₁₂-Alkyl oder Halogen, Benzyl oder Benzyl, substituiert mit C₁-C₁₂-Alkyl oder Halogen bedeutet; C₁-C₁₂-Alkoxyacetyl oder Phenoxyacetyl, das unsubstituiert oder mit linearem oder verzweigten C₁-C₆-Alkyl, C₁-C₆-Halogenalkyl, Halogen, C₁-C₆-Alkoxy, C₁-C₆-Alkylthio , Nitro oder Cyano ein- oder mehrfach substituiert sein kann.

Beispiele für C₁-C₈-Alkyl sind Methyl, Ethyl, n- und i-Propyl, n-, i-und t-Butyl; für ein monocyclisches C₇-C₁₂-Aralkyl sind Benzyl, Methylbenzyl, Dimethylbenzyl; für ein mono-oder bicyclisches C₇-C₁₂-Aralkoxy sind Methoxybenzyl, Dimethoxybenzyl; für ein mono- oder bicyclisches C₇-C₁₂-Haloalkyl ist Brombenzyl; für ein substituiertes Diphenylmethyl sind Di(methylphenyl)methyl, Di(dimethylphenyl)methyl, Di(methoxyphenyl)methyl, Di(dimethoxyphenyl)methyl; für ein substituiertes Triphenylmethyl sind Tri(methylphenyl)methyl, Tri(dimethylphenyl)methyl, Tri(methoxyphenyl)methyl, Tri(dimethoxyphenyl)methyl, Monomethoxytrityl, Dimethoxytrityl und tris-p-tert.-Butylphenylmethyl; für Silylgruppen sind Triphenylsilyl, Trimethylsilyl, Triethylsilyl, Tri-n-propylsilyl, i-Propyl-dimethylsilyl, t-Butyl-dimethylsilyl, t-Butyl-diphenylsilyl, n-Octyl-dimethylsilyl, (1,1,2,2-Tetramethylethyl)-dimethylsilyl; für die Gruppe R-C(O)- sind Acetyl, Trifluoracetyl , Propanoyl, Butanoyl, Pentanoyl, Hexanoyl, Benzoyl, Methylbenzoyl, Methoxybenzoyl, Chlorbenzoyl, Brombenzoyl, Methoxy-, Ethoxy-, n- oder i-Propoxy- oder n-, i- oder t-Butoxycarbonyl, oder Phenyloxycarbonyl, Benzyloxycarbonyl, Methyl- oder Methoxy-oder Chlorphenyloxycarbonyl oder -benzyloxycarbonyl oder 9-Fluorenylmethyloxycarbonyl; und für die Gruppe R₁₇-SO₂- sind Methyl-, Ethyl-, Propyl-, Butyl-, Phenyl-, Benzyl-, p-Brom-, p-Methoxy- und p-Methylphenylsulfonyl; und für Alkoxyacetyl und Phenoxyacetyl Methoxyacetyl, Ethoxyacetyl, Phenoxyacetyl, (p-Methylphenoxy)acetyl, (p-Tertiärbutylphenoxy)acetyl. In Nukleinsäurebasen werden auch häufig Amidinschutzgruppen verwendet.

Die Nukleoside und Oligonukleotide können kovalent über eine Brückengruppe an ein festes Trägermaterial gebunden sein. Geeignete Trägermaterialien sind zum Beispiel Kieselgele, Controlled Pore Glass, Polystyrol, Polyacrylamid, Polyurethane, Polyolefine, Melamin, Polyamide, Polester, Polyether, Polyalkohole, Cyclodextrin, Cellulose und veretherte oder acylierte Cellulosederivate, Glykogen, Stärke, Chitin und Chitosan. Die Brückengruppe kann je nach Wahl des Trägermaterials zum Beispiel von Dicarbonsäuren, Diurethanen oder Alkoxysilylurethanen abgeleitet sein.

Das erfindungsgemässe Verfahren kann gegebenenfalls in Gegenwart eines zusätzlichen Lösungsmittels durchgeführt werden. Ohne zusätzliches Lösungsmittel ist der im erfindungsgemässen Verfahren verwendete Alkohol, das Polyol oder das polymere Polyol, sofern flüssig, das Lösungsmittel. Als zusätzliche Lösungsmittel eignen sich solche Lösungsmittel, die im jeweiligen Reaktionsansatz weder mit der Base noch mit dem Alkohol, dem Polyol oder dem polymeren Polyol konkurrieren. Sie können allein oder in Mischungen aus mindestens zwei Lösungsmitteln verwendet werden. Bei Polyolen können auch Lösungsmittel oder Lösungszusätze verwendet werden, welche durch Quellen des Polyols die Alkoholgruppen dem erfindungsgemässen Verfahren zugänglich machen. Beispiele für derartige Lösungsmittel sind ausgewählt aus der Gruppe bestehend aus Ethern, halogenierten Kohlenwasserstoffen, Carbonsäureamiden, Lactamen, Sulfoxiden, Sulfonen; aromatischen Kohlenwasserstoffen, Nitrilen, aliphatischen und cycloaliphatischen Kohlenwasserstoffen, insbesondere Ether, halogenierte Kohlenwasserstoffe, Nitrile, Carbonsäureamide und Lactame. Lösungszusätze im Rahmen der vorliegenden Erfindung können vorteilhafterweise Salze, beispielsweise Ammoniumacetate und Ammoniumhydrogencarbonate, sein.

Besonders geeignete Ether sind Dibutylether, Tetrahydrofuran, Dioxan, Ethylenglykoldiethyl - und -dimethylether, Diethylenglykoldimethylether, Diethylenglykoldiethylether oder Triethylenglykoldimethylether, insbesondere Methyl-t-butylether, Dibutylether, Diisopropylether, Tetrahydrofuran oder Dioxan. Besonders geeignete halogenierte Kohlenwasserstoffe sind Methylenchlorid, Chloroform, 1,2-Dichlorethan, 1,1,1-Trichlorethan und 1,1,2,2-Tetrachlorethan.

Von den Amiden werden vorteilhafterweise N-alkylierte oder N-dialkylierte Amide verwendet. Typische Vertreter sind beispielsweise N,N-Dimethylformamid, N,N-Diethylformamid, N,N-Dimethylacetamid, Tetramethylharnstoff und Hexamethylphosphorsäuretriamid

Bevorzugte Lactame sind ausgewählt aus der Gruppe bestehend aus γ-Butyrolactam, ε-Caprolactam, N-Methylpyrrolidon, N-Acetylpyrrolidon und N-Methylcaprolactam.

Weitere geeignete Lösungsmittel im Rahmen der vorliegenden Erfindung sind beispielsweise Dimethylsulfoxid, Dimethylsulfon, Diethylsulfon, Trimethylensulfon, Tetramethylensulfon, Benzol, substituiertes Benzol, insbesondere Chlorbenzol, o-Dichlorbenzol, 1,2,4-Trichlorbenzol, Nitrobenzol, Toluol und Xylol, Acetonitril, Propionitril, Benzonitril, Phenylacetonitril, Pentan, Petrolether, Hexan, Cyclohexan und Methylcyclohexan.

Der im erfindungsgemässen Verfahren verwendete aliphatische, cycloaliphatische, araliphatische oder aromatische Alkohol, das Polyol oder das polymere Polyol werden nachfolgend als Akzeptor bezeichnet.

Vorteilhafterweise wird ein Alkanol mit 1 bis 20 Kohlenstoffatomen, bevorzugt mit 1 bis 12 Kohlenstoffatomen, insbesondere mit 1 bis 6 Kohlenstoffatomen verwendet, beispielsweise ein Vertreter der Gruppe bestehend aus Methanol, Ethanol, n- und i-Propanol, n-, i- und t-Butanol, Pentanol, Hexanol, Ethylenglykol-, Diethylenglykolmonomethyl-und -monoethylether. Eine bevorzugte Untergruppe der im erfindungsgemässen Verfahren verwendeten Alkohole sind C₁-C₄-Alkanole. Besonders bevorzugt wird Methanol verwendet.

Des weiteren eignet sich ein gegebenenfalls alkylsubstituiertes Cycloalkanol mit 5 bis 12 Ringkohlenstoffatomen, vorzugsweise mit 5 bis 8 Ringkohlenstoffatomen, beispielsweise ausgewählt aus der Gruppe bestehend aus Cyclopentanol, Cyclohexanol, Methylcyclohexanol, Cycloheptanol und Cyclooctanol.

Beispiele für weitere geeignete Akzeptoren sind araliphatische Alkohole mit 7 bis 30 Kohlenstoffatomen, insbesondere mit 7 bis 20 Kohlenstoffatomen, vorzugsweise ausgewählt aus der Gruppe bestehend aus un-, Alkyl- oder Alkoxy-substituiertem Benzylalkohol und β-Phenylethanol; aromatische Alkohole mit 6 bis 30 Kohlenstoffatomen, insbesondere mit 6 bis 12 Kohlenstoffatomen, vorzugsweise ausgewählt aus der Gruppe bestehend aus Phenol und un-, Alkyl- oder Alkoxy-substituiertes Naphthol; Polyole mit 2 bis 20 Kohlenstoffatomen, vorzugsweise ausgewählt aus der Gruppe bestehend aus Ethylenglykol, Propylenglykol, Butandiol, Pentandiol, Hexandiol, Cyclohexandiol, Dihydroxymethylcyclohexan, Diethylenglykol, Triethylenglykol, Polyethylenglykol, Trimethylolpropan, Pentaerythrit, Dipentaerythrit, Glycerin und Saccharide; polymere Polyole, vorzugsweise ausgewählt aus der Gruppe bestehend aus Polyvinylalkohol, Polysaccharide und Polyacryl-bzw. -methacrylsäurehydroxyalkylester. Die Substituenten Alkyl und Alkoxy enthalten bevorzugt 1 bis 4 Kohlenstoffatome.

Im Rahmen der vorliegenden Erfindung werden unter Sacchariden als Akzeptoren beispielsweise Mono- und Oligosaccaride wie Mono-, Di-, Tri-, Tetra- und Pentasaccharide verstanden. In einer bevorzugten Ausführungsform handelt es sich bei den Mono-und Oligosacchariden um Aldosen oder Ketosen. In einer besonders bevorzugten Ausführungsform handelt es sich bei dem Monosaccharid um Aldopentosen, Aldohexosen, Ketopentosen oder Ketohexosen.

Beispiele für eine Aldopentose sind D-Ribose, D-Arabinose, D-Xylose oder D-Lyxose; für eine Aldohexose D-Allose, D-Altrose, D-Glucose, D-Mannose, D-Gulose, D-Idose, D-Galaktose oder D-Talose; für eine Ketopentose D-Ribulose oder D-Xylulose; für eine Ketohexose D-Psicose, D-Fructose, D-Sorbose oder D-Tagatose.

Beispiele für ein Disaccharid sind Trehalose, Maltose, Isomaltose, Cellobiose, Gentibiose, Saccharose oder Lactose.

Als Trisaccharid sei beispielhaft Raffinose genannt.

Beispiele für Polysaccharide sind Cellulose, Stärke, Dextrane, Glykogen, Fructane, Inulin, Mannane, Xylane und Arabinane.

Bei der im Rahmen der vorliegenden Erfindung verwendeten anorganischen Base handelt es sich vorzugsweise um einen Vertreter der Gruppe bestehend aus basischem Metalloxid, Schwermetalloxid und -hydroxid, bevorzugt um SiO₂ oder basisches Aluminiumoxid.

Weitere geeignete anorganische Basen sind solche, die die Umesterung nukleophil katalysieren können und Bestandteil eines Puffersystems sein können. Vorzugsweise handelt es sich um in Alkoholen oder dem Reaktionsgemisch lösliche Metall- oder Ammoniumfluoride, Metall- oder Ammoniumaldoximate, -ketoximate, -hydroxomate, -sulfate, -phosphate, -phosphonate, -phophosphate, -sulfamate, -nitite, -nitrate, -sulfonate, wobei Metall vorzugsweise für ein Alkalimetallion steht, zum Beispiel Na⁺, Li⁺, K⁺, Rb⁺ und Cs⁺. Die Metallaldoximate, -ketoximate, -hydroxomate, -sulfamate, -sulfonate und phosphonate können H-Atome oder unsubstituierte oder mit Halogen, C₁-C₄-Alkyl, C₁-C₄-Alkoxy, Cyano oder Nitro substituierte C₁-C₆-Alkylgruppen oder Phenylgruppen enthalten. Bei den Ammoniumfluoriden handelt es sich bevorzugt um Tetra(C₁-C₆-Alkyl)ammoniumfluorid, zum Beispiel Tetra(n-butyl)ammoniumfluorid.HF. Weitere Beispiele sind CsF, KF, CH₃-C(O)-NH-O⁻ und Mg(CF₃CO)₂. Es hat sich als besonders zweckmässig erwiesen, die nukleophilen Basen gemeinsam mit den organischen Stickstoffbasen zu verwenden, vorteilhaft in katalytischen Mengen.

Als organische Stickstoffbase eignet sich vorteilhafterweise ein Vertreter der Gruppe bestehend aus aromatischem Amin, aromatischem N-Heterocyclus und N-C₁-C₄-Alkylmorpholin. Als typische Vertreter sind zu nennen Imidazol, Pyridin, N-Methylmorpholin, Anilin und o-Diaminobenzol, sowie Polyvinylpyridin.

Die im erfindungsgemässen Verfahren eingesetzte Menge Base reicht von einer katalytischen Menge bis zu höheren Mengen, beispielsweise bis zu äquivalenten Mengen oder einem Überschuss. Eine katalytische Menge im Rahmen der vorliegenden Erfindung liegt im Bereich von 0,01 bis 20 Mol %, vorteilhafterweise von 0,1 bis 10 Mol %, insbesondere von 0,1 bis 5 Mol %, bezogen auf 1 Mol Nukleosid bzw. Oligonukleotid.

Im erfindungsgemässen Verfahren wird vorzugsweise ein Überschuss von mindestens 100 Moläquivalenten und bis zu 1000 Moläquivalenten oder mehr Hydroxylgruppen des Akzeptors pro Moläquivalent Gruppen der Formel I eingesetzt.

Das erfindungsgemässe Verfahren kann durch die zusätzliche Verwendung von Lewissäuren in Mengen von vorzugsweise 0,01 bis 20 Mol %, vorteilhafterweise von 0,1 bis 10 Mol %, insbesondere von 0,1 bis 5 Mol %, bezogen auf 1 Mol Nukleosid bzw. Oligonukleotid wesentlich beschleunigt werden. Die erzielbare hohe Reaktionsgeschweindigkeit ist ein erheblicher Vorteil. Als Lewissäuren können zum Beispiel Metallsalze (LiCl), Borsäureester oder organische Metallkomplexe verwendet werden. Die gemeinsame Verwendung von organischer Stickstoffbase, nukleophile anorganische Base und Lewissäure im erfindungsgemässen Verfahren ist besonders vorteilhaft, da eine hohe Selektivität erzielt wird.

Das Verfahren wird vorteilhafterweise bei einer Temperatur von -20°C bis 55°C, besonders bevorzugt bei einer Temperatur von 15°C bis 35°C, insbesondere bei Raumtemperatur durchgeführt.

Das erfindungsgemässe Verfahren kann zum Beispiel so durchgeführt werden, dass man zu einer Lösung oder Mischung aus Alkohol und Base ein Nukleosid oder Oligonukleotid mit Resten der Formeln I, Ia und Ib gibt und dieses Reaktionsgemisch ausreagieren lässt. Zur Isolierung der gewünschten Verbindungen können zum Beispiel gegebenenfalls zunächst unlösliche Bestandteile abfiltriert und dann das Lösungsmittel verdampft werden. Der Rückstand kann mit üblichen Methoden wie zum Beispiel Kristallisation oder Chromatographie gereinigt werden. In einer besonders vorteilhaften Ausgestaltung des erfindungsgemässen Verfahrens lässt man Abflusslösungen der Oligonukleotidsynthese direkt in eine Lösung oder Mischung aus Alkohol und Base laufen, um die im grossen Überschuss verwendeten Nukleosidbausteine zurückzugewinnen. Bei dieser Vorgehensweise wird die Abflusslösung, die eine Verbindung mit einem Rest der Formeln I, Ia und Ib enthält, in die Reaktionslösung oder das Reaktionsgemisch überführt, wobei zu Beginn der Reaktion ein sehr grosser Basenüberschuss auftritt und saure Bestandteile solcher Abflusslösungen neutralisiert werden können. Im Verlauf der Reaktion nimmt die Basenmenge ab und kann gegebenenfalls ersetzt werden. Diese sauren Bestandteile können vorzugsweise Tetrazol oder andere Säuren sein, die die Nukleotidkondensation gemäss des Phosphittriesterverfahrens bewirken.

Zur Aufarbeitung der Reaktionsgemische können die gebildeten Phosphite mit üblichen Oxidationsmitteln, zum Beispiel I₂/H₂O/Pyridin oder S₈/Pyridin zu den entsprechenden Phosphaten bzw. Thiophosphaten aufgearbeitet werden. Beim Eindampfen der Reaktionslösungen kann dann keine Rückreaktion mehr stattfinden und die Isolierung der gewünschten Produkte kann oft unter Vermeidung chromatographischer Methoden durch Extraktion erfolgen.

Es hat sich als zweckmässig erwiesen, die verwendete organische Stickstoffbase, zum Beispiel Imidazol, mit einer Citratpufferlösung (pH etwa 3 bis 4) mittels Extraktion zu entfernen. Hiermit erzielt man eine praktisch vollständige Abtrennung und eine gute Phasentrennung bei einer wässrigen Aufarbeitung.

In einer besonderen Ausführungsform wird Cyanoethanol als Akzeptor eingesetzt, wenn die Nukleoside bzw. Oligonukleotide an ein festes Trägermaterial gebunden sind und eine H-Phosphonat- oder H-Phosphinatgruppe enthalten und in Anwesenheit einer organischen Stickstoffbase umgesetzt werden. Hierbei werden Biscyanoethylphosphit bzw. Cyanoethyl-methylphosphinat gebildet, die mit schwach basischen polymeren Ionenaustauschem irreversibel umgesetzt werden. Die entstehenden Phosphorverbindung bleiben durch Salzbildung am Ionenaustauscher gebunden und gleichzeitig wird das gebildete Acrylnitril vom Ionenaustauscher abgefangen. Mit dieser Ausführungsform wird eine Rückreaktion während des Umesterungsverfahrens vermieden und es können besonders hohe Akzeptorüberschüsse vermieden werden.

Die nachfolgenden Beispiele erläutern die Erfindung näher. Die in den Beispielen 2, 5 und 7 verwendeten Abflusslösungen werden folgendermassen erhalten: Die Abflusslösungen eines Festphasenoligonukleotidsynthesereaktors werden gemäss des Verfahrens von Gao et al. (Gao, H., Gaffney, B.L., Jones, R.A., Tetrahedron Lett., im Druck, 1991) aufgefangen. Die Abflusslösungen fliessen direkt nach dem Austritt aus dem Reaktor nach Nukleosiden getrennt in eine Lösung aus 50 ml Methanol/Acetonitril 2:1.

### Beispiel 1: Darstellung von 5'-Dimethoxytritylthymidin aus Dimethoxytritylthymidin-3'yl-β-cyanoethylphosphordiisopropylamidit

Zu einer Lösung aus 360 mg (0,48 mMol) Dimethoxytritylthymidin-3'-yl-β-cyanoethylphosphordiisopropylamidit in 4 ml Acetonitril werden 270 mg (3,86 mMol) Tetrazol und 9 µl (0,5 mMol) Wasser hinzugegeben. Nach 5 Minuten ist die Hydrolyse des Phosphoramidits abgeschlossen und man erhält das 5'-Dimethoxytritylthymidin-3'-cyanoethyl-hydrogenphosphonat.

Dieser Lösung werden 4 ml Methanol und 350 mg Imidazol zugesetzt. Nach 24 Stunden wird die Reaktionslösung im Vakuum eingampft. Man erhält einen weissen bis schwach gelben Feststoff, der in 50 ml Dichlormethan aufgenommen wird.

Es folgen Extraktionen mit je 50 ml Wasser und Natriumhydrogencarbonat. Die organische Phase wird über Natriumsulfat getrocknet. Man filtriert das Salz und eventuelle Imidazolreste ab. Das Rohprodukt wird eingedampft und durch Flashchromatographie (Still, W.C., Kahn, M., Mitra, A., J. Org. Chem. 43:2923, 1978) aufgetrennt. Die Produktfraktionen werden vereinigt und eingeengt. Anschliessend werden sie mit 50 ml Dichlormethan aufgenommen und mit 50 ml 1 M Triethylammonium-hydrogencarbonat extrahiert. Die organische Phase wird über Natriumsulfat getrocknet. Das Salz wird abfiltriert und auf 1 ml im Vakuum eingeengt. Die konzentrierte Produktlösung wird bei Raumtemperatur in 50 ml n-Pentan ausgefällt. Die Fällung wird durch Zugabe von 200 ml n-Pentan und nachfolgendem Abkühlen auf -20°C vervollständigt. Der Niederschlag wird abfiltriert, mit 50 ml n-Pentan gewaschen und im Vakuum über P₄O₁₀KOH getrocknet. Das erhaltene Produkt ist 5'-Dimethoxytritylthymidin (5'-Dimethoxytritylthymidin: 431 mg, 82,5 % der Trockenmasse).

### Beispiel 2: Darstellung von 5'-Dimethoxytritylthymidin aus der Abflusslösung eines Oligonukleotidsynthesereaktors

In 50 ml einer Lösung aus 1 g Imidazol in Methanol laufen 100 ml einer im Verlauf der DNA-Synthese anfallenden Abflusslösung des Reaktors ein, die 0,24 mMol 5'-Dimethoxytritylthymidin-3'-yl-β-cyanoethylphosphonat enthält. Nach Abschluss der Reaktion wird die Reaktionslösung im Vakuum getrocknet. Man erhält einen weissen bis schwach gelben Feststoff, der in 100 ml Dichlormethan aufgenommen wird. Es folgen drei Extraktionen mit je 100 ml 1 M NaH₂PO₄/NaHPO₄, pH 5,5. Die vereinigte wässrige Phase wird mit 50 ml Dichlormethan extrahiert. Die organischen Phasen werden vereinigt und anschliessend zwei Extraktionen mit je 100 ml gesättigter wässriger Natriumhydrogencarbonatlösung unterworfen. Die Natriumhydrogencarbonatphase wird mit 50 ml Dichlormethan extrahiert und alle organischen Phasen vereinigt. Die vereinigte organische Phase wird über Natriumsulfat getrocknet. Es folgt eine Umkristallisation aus 5 ml Benzol, die durch Abkühlen auf -20°C vervollständigt wird. Anschliessend werden 50 ml n-Hexan zugesetzt und die Kristallisation bei -20°C für 20 Stunden weitergeführt. Man filtriert den Niederschlag ab, wäscht ihn mit 50 ml n-Hexan und trocknet ihn anschliessend im Vakuum über P₄O₁₀/KOH. Das erhaltene Produkt ist 5'-Dimethoxytritylthymidin (5'-Dimethoxytritylthymidin: 131 mg, 99 % der Trockenmasse bei 100 % Internukleotidkopplung im Synthesizer).

### Beispiel 3: Darstellung von 2-i-Butyryl-5'-dimethoxytrityl-2'-desoxyguanosin aus 2-i-Butyryl-5'-dimethoxytrityl-2'-desoxyguanosin -3'-yl-β-cyanoethylphosphordiisopropylamidit

Zu einer Lösung aus 839 mg (1 mMol) 2-i-Butyryl-5'-dimethoxytrityl-2'-desoxyguanosin-3'-yl-β-cyanoethylphosphordiisopropylamidit in 8 ml Acetonitril werden 350 mg (5 mMol) Tetrazol und 18 µl (1 mMol) Wasser hinzugegeben. Nach 5 Minuten ist die Hydrolyse des Phosphoramidits abgeschlossen und man erhält das 5'-Dimethoxytritylguanosin3'-cyanoethyl-hydrogenphosphonat.

Dieser Lösung werden 4 ml Methanol und 350 mg Imidazol zugesetzt. Nach 24 Stunden wird die Reaktionslösung im Vakuum eingedampft. Man erhält einen weissen bis schwach gelben Feststoff, der in 50 ml Dichlormethan aufgenommen wird.

Es folgen Extraktionen mit je 50 ml Wasser und Natriumhydrogencarbonat. Die organische Phase wird über Natriumsulfat getrocknet. Man filtriert das Salz und eventuelle Imidazolreste ab. Das Rohprodukt wird eingedampft und durch Flaschromatographie aufgetrennt. Die Produkfraktionen werden vereinigt und eingeengt. Anschliessend werden sie mit 50 ml Dichlormethan aufgenommen und mit 50 ml 1 M Triethylammonium-hydrogencarbonat extrahiert. Die organische Phase wird über Natriumsulfat getrocknet. Das Salz wird abfiltriert und auf 1 ml im Vakuum eingeengt. Die konzentrierte Produktlösung wird bei Raumtemperatur in 50 ml n-Pentan ausgefällt. Die Fällung wird durch Zugabe von 450 ml n-Pentan und nachfolgendem Abkühlen auf -20°C vervollständigt. Der Niederschlag wird abfiltriert, mit 100 ml n-Pentan gewaschen und im Vakuum über P₄O₁₀/KOH getrokknet. Das erhaltene Produkt ist 2-i-Butyryl-5'-dimethoxytrityl-2'-desoxyguanosin (2-i-Butyryl-5'-dimethoxytrityl-2'-desoxyguanosin: 577 mg, 90,3 % der Trockenmasse).

### Beispiel 4: Darstellung von 6-Benzoyl-5'-dimethoxytrityl-2'-desoxyadenosin aus 6-Benzoyl-5'-dimethoxytrityl-2'-desoxyadenosin-3'-yl-β-cyanoethylphosphordiisopropylamidit

Zu einer Lösung aus 857 mg (1 mMol) 6-Benzoyl-5'-dimethoxytrityl-2'-desoxyadenosin-3'-yl-β-cyanoethylphosphordiisopropylamidit in 8 ml Acetonitril werden 350 mg (5 mMol) Tetrazol und 18 µl (1 mMol) Wasser hinzugegeben. Nach 5 Minuten ist die Hydrolyse des Phosphoramidits abgeschlossen und man erhält das 5'-Dimethoxytrityladenosin-3'-cyanoethyl-hydrogenphosphonat.

Dieser Lösung werden 4 ml Methanol und 350 mg Imidazol zugesetzt. Nach 24 Stunden wird die Reaktionslösung im Vakuum eingedampft. Man erhält einen weissen bis schwach gelben Feststoff, der in 50 ml Dichlormethan aufgenommen wird.

Es folgen Extraktionen mit je 50 ml Wasser und Natriumhydrogencarbonat. Die organische Phase wird über Natriumsulfat getrocknet Man filtriert das Salz und eventuelle Imidazolreste ab. Das Rohprodukt wird eingedampft und durch Flashchromatographie aufgetrennt. Die Produktfraktionen werden vereinigt und eingeengt. Anschliessend werden sie mit 50 ml Dichlormethan aufgenommen und mit 50 ml 1 M Triethylammonium-hydrogencarbonat extrahiert. Die organische Phase wird über Natriumsulfat getrocknet. Das Salz wird abfiltriert und auf 1 ml im Vakuum eingeengt. Die konzentrierte Produktlösung wird bei Raumtemperatur in 50 ml n-Pentan ausgefällt. Die Fällung wird durch Zugabe von 450 ml n-Pentan und nachfolgendem Abkühlen auf -20°C vervollständigt. Der Niederschlag wird abfiltriert, mit 100 ml n-Pentan gewaschen und im Vakuum über P₄O₁₀/KOH getrokknet. Das erhaltene Produkt ist 6-Benzoyl-5'-dimethoxytrityl-2'-desoxyadenosin-(6-Benzoyl-5'-dimethoxytrityl-2'-desoxyadenosin: 562 mg, 85,5 % der Trockenmasse).

### Beispiel 5: Darstellung von 6-Benzoyl-5'-dimethoxytrityl-2'-desoxyadenosin aus der Abflusslösung eines Oligonukleotidsynthesereaktors

In 50 ml einer Lösung aus 1 g Imidazol in Methanol laufen 300 ml einer im Verlauf der DNA-Synthese anfallenden Abflusslösung des Reaktors ein, die 0,82 mMol 6-Benzoyl-5'-dimethoxytrityl-2'-desoxyadenosin-3'-yl-β-cyanoethylphosphonat enthält. Nach Abschluss der Reaktion wird die Reaktionslösung im Vakuum getrocknet. Man erhält einen weissen bis schwach gelben Feststoff, der in 100 ml Dichlormethan aufgenommen wird. Es folgen drei Extraktionen mit je 50 ml 1 M NaH₂PO₄/NaHPO₄, pH 5,5. Die vereinigte wässrige Phase wird mit 50 ml Dichlormethan extrahiert. Die organischen Phasen werden vereinigt und anschliessend zwei Extraktionen mit je 100 ml gesättigter wässriger Natriumhydrogencarbonatlösung unterworfen. Die Natriumhydrogencarbonatphase wird mit 50 ml Dichlormethan extrahiert und alle organischen Phasen vereinigt. Die vereinigte organische Phase wird über Natriumsulfat getrocknet. Das Rohprodukt wird eingedampft und durch Flashchromatographie aufgetrennt. Die Produktfraktionen werden vereinigt und eingeengt. Anschliessend werden sie mit 50 ml Dichlormethan aufgenommen und mit 50 ml 1 M Triethylammonium-hydrogencarbonat extrahiert. Die organische Phase wird über Natriumsulfat getrocknet. Das Salz wird abfiltriert und auf 1 ml im Vakuum eingeengt. Die konzentrierte Produktlösung wird bei Raumtemperatur in 50 ml n-Pentan aüsgefällt. Die Fällung wird durch Zugabe von 200 ml n-Pentan und nachfolgendem Abkühlen auf -20°C vervollständigt. Der Niederschlag wird abfiltriert, mit 50 ml n-Pentan gewaschen und im Vakuum über P₄O₁₀/KOH getrocknet. Das erhaltene Produkt ist 6-Benzoyl-5'-dimethoxytrityl-2'-desoxyadenosin(6-Benzoyl-5'-dimethoxytrityl-2'-desoxyadenosin: 380 mg, 75,5 % der Trockenmasse bei 100 % Internukleotidkopplung im Synthesizer).

### Beispiel 6: Darstellung von 4-Benzoyl-5'-dimethoxytrityl-2'-desoxycytidin aus 4-Benzoyl-5'-dimethoxytrityl-2'-desoxycytidin-3'-yl-β-cyanoethylphosphordiisopropylamidit

Zu einer Lösung aus 833 mg (1 mMol) 4-Benzoyl-5'-dimethoxytrityl-2'-desoxycytidin-3'-yl-β-cyanoethylphosphordiisopropylamidit in 8 ml Acetonitril werden 350 mg (5 mMol) Tetrazol 1 und 18 µl (1 mMol) Wasser hinzugegeben. Nach 5 Minuten ist die Hydrolyse des Phosphoramidits abgeschlossen und man erhält das 5'-Dimethoxytritylcytidin-3'-cyanoethyl-hydrogenphosphonat.

Dieser Lösung werden 4 ml Methanol und 350 mg Imidazol zugesetzt. Nach 24 Stunden wird die Reaktionslösung im Vakuum eingedampft. Man erhält einen weissen bis schwach gelben Feststoff, der in 50 ml Dichlormethan aufgenommen wird.

Es folgen Extraktionen mit je 50 ml Wasser und Natriumhydrogencarbonat. Die organische Phase wird über Natriumsulfat getrocknet. Man filtriert das Salz und eventuelle Imidazolreste ab. Das Rohprodukt wird eingedampft und durch Flashchromatographie aufgetrennt. Die Produktfraktionen werden vereinigt und eingeengt. Anschliessend werden sie mit 50 ml Dichlormethan aufgenommen und mit 50 ml 1 M Triethylammonium-hydrogencarbonat extrahiert. Die organische Phase wird über Natriumsulfat getrocknet. Das Salz wird abfiltriert und auf 1 ml im Vakuum eingeengt. Die konzentrierte Produktlösung wird bei Raumtemperatur in 50 ml n-Pentan ausgefällt. Die Fällung wird durch Zugabe von 450 ml n-Pentan und nachfolgendem Abkühlen auf -20°C vervollständigt. Der Niederschlag wird abfiltriert, mit 100 ml n-Pentan gewaschen und im Vakuum über P₄O₁₀/KOH getrokknet. Das erhaltene Produkt ist 4-Benzoyl-5'-dimethoxytrityl-2'-desoxycytidin (4-Benzoyl-5'-dimethoxytrityl-2'-desoxycytidin: 527 mg, 83,2 % der Trockenmasse).

### Beispiel 7: Darstellung von 4-Benzoyl-5'-dimethoxytrityl-2'-desoxycytidin aus der Abflusslösung eines Oligonukleotidsynthesereaktors

In 50 ml einer Lösung aus 1 g Imidazol in Methanol laufen 100 ml einer im Verlauf der DNA-Synthese anfallenden Abflusslösung des Reaktors ein, die 0,27 mMol 4-Benzoyl-5'-dimethoxytrityl-2'-desoxycytidin-3'-yl-β-cyanoethylphosphonat enthält. Nach Abschluss der Reaktion wird die Reaktionslösung im Vakuum getrocknet. Man erhält einen weissen bis schwach gelben Feststoff, der in 100 ml Dichlormethan aufgenommen wird. Es folgen drei Extraktionen mit je 50 ml 1 M NaH₂PO₄/NaHPO₄, pH 5,5. Die vereinigte wässrige Phase wird mit 50 ml Dichlormethan extrahiert. Die organischen Phasen werden vereinigt und anschliessend zwei Extraktionen mit je 100 ml gesättigter wässriger Natriumhydrogencarbonatlösung unterworfen. Die Natriumhydrogencarbonatphase wird mit 50 ml Dichlormethan extrahiert und alle organischen Phasen vereinigt. Die vereinigte organische Phase wird über Natriumsulfat getrocknet. Das Rohprodukt wird eingedampft und durch Flashchromatographie aufgetrennt. Die Produktfraktionen werden vereinigt und eingeengt. Anschliessend werden sie mit 50 ml Dichlormethan aufgenommen und mit 50 ml 1 M Triethylammonium-hydrogencarbonat extrahiert. Die organische Phase wird über Natriumsulfat getrocknet. Das Salz wird abfiltriert und auf 1 ml im Vakuum eingeengt. Die konzentrierte Produktlösung wird bei Raumtemperatur in 50 ml n-Pentan ausgefällt. Die Fällung wird durch Zugabe von 200 ml n-Pentan und nachfolgendem Abkühlen auf - 20°C vervollständigt. Der Niederschlag wird abfiltriert, mit 50 ml n-Pentan gewaschen und im Vakuum über P₄O₁₀/KOH getrocknet. Das erhaltene Produkt ist 4-Benzoyl-5'-dimethoxytrityl-2'-desoxycytidin (4-Benzoyl-5'-dimethoxytrityl-2'-desoxycytidin: 163 mg, 91 % der Trockenmasse bei 100 % Internukleotidkopplung im Synthesizer).

### Beispiel 8: Darstellung von N-Methyl,N-(5'-Dimethoxytrityl-3'- deoxythymid-3'-yl)-(5'-deoxythymid-5'-yl)acetamid aus der Abflusslösung eines Oligonucleotidsynthesereaktors.

In 50 ml einer Lösung aus 1 g Imidazol in Methanol laufen 5 ml einer im Verlauf der DNA-Synthese anfallenden Abflusslösung des Reaktors ein, die 160 mg N-Methyl,N-(5'-Dimethoxytrityl-3'-deoxythymid-3'-yl)-(3'cyanoethyl-H-phosphonyl-5'-deoxythymid-5'-yl)acetamid enthält. Man lässt drei Tage bei 20-25°C reagieren. Die Reaktionsmischung wird mit 5ml Wasser versetzt. Anschliessend wird eine so grosse Menge einer Lösung aus 30 g Jod, 20 ml Wasser, 200ml Pyridin und 750 ml Tetrahydrofuran der Reaktionsmischung zugegeben, bis die braune Jodfarbe für mindestens 5 Minuten bestehen bleibt. Die Reaktionsmischung wird bis zu einem gelblichen Feststoff im Vakuum eingedamft. Man nimmt in einer Emulsion aus 100 ml 40% wässriger NaHSO₃ und 50 ml Ethylacetat auf. Man trennt die wässrige Phase ab und extrahiert die organische Phase erneut zwei mal mit je 50 ml wässriger Natriumhydrogencarbonat Lösung. Die wässrigen Phasen werden vereinigt und erneut mit 10 ml Ethylacetat exrahiert. Die organischen Phasen werden vereinigt, 2mal mit je 50 ml 1M wässrigem MgSO₄ extrahiert und anschliessend über 2g trokkenem Natriumsulfat getrocknet. Nach dem Abfiltrieren des Salzes wird das Filtrat im Vakuum zur Trockne eingeengt. Man nimmt den Feststoff in 5ml CH-Cl₃/Methanol(9:1) auf und chromatographiert über einer Kieselgelsäule. Die Produktfraktionen werden vereinigt und am Hochvakuum getrocknet. Das Produkt N-Methyl,N-(5'-Dimethoxytrityl-3'-deoxythymid-3'-yl)-(5'-deoxythymid-5'-yl)acetamid fällt als weisser Schaum in einer Ausbeute von 40 mg (31 % d. Th.) an.
¹H-NMR: (CDCl₃, Standard = TMS, d[ppm]): 8,8: 2s, NH; 7,7: s, 1H, H⁶; 7,45-7,2: DMTr; 7,09: s, 1H, H⁶; 6,82: 2m, 4H, DMTr; 6,33: t, 1H, 1'; 6,15: t,1H, 1'; 5,26, 4,95: m, 1H, 3' am NRCH₃, 2 Rotamere 3:1; 4,25-4: m, 2H; 3,75: s, 6H, DMTr; 3,63, 3,4, 3,25: 3m, 4H; 2,9, 2,85: 2s, 3H, CH₃ von NRCH₃, 2 Rotamere: 3:1; 2,65-2: m; 1,88: 2H, 1,8: 1H, 2s, CH₃⁵, 2 Rotamere: 3:1; 1,5: 1H, 1,45; 2H, 2s, CH₃⁵, 2 Rotamere: 1:3.

### Beispiel 9: Darstellung von 5'-Dimethoxytrityl-N⁶-pivaloyl-2'(-1-(2-fluorophenyl)-4-methoxy-piperid-4-yl)-adenosin aus der Abflusslösung eines Oligonucleotidsynthesereaktors.

In 50 ml einer Lösung aus 1 g Imidazol in Methanol laufen 5 ml einer im Verlauf der DNA-Synthese anfallenden Abflusslösung des Reaktors ein, die 160 mg N-Methyl,N-(5'-Dimethoxytrityl-3'-deoxythymid-3'-yl)-(3'cyanoethyl-H-phosphonyl-5'-deoxythymid-5'-yl)acetamid enthält. Man lässt drei Tage bei 20-25°C reagieren. Die Reaktionsmischung wird mit 5ml Wasser versetzt. Anschliessend wird eine so grosse Menge einer Lösung aus 30 g Jod, 20 ml Wasser, 200ml Pyridin und 750 ml Tetrahydrofuran der Reaktionsmischung zugegeben, bis die braune Jodfarbe für mindestens 5 Minuten bestehen bleibt. Die Reaktionsmischung wird bis zu einem gelblichen Feststoff im Vakuum eingedamft. Man nimmt in einer Emulsion aus 100 ml 40% wässriger NaHSO₃ und 50 ml Ethylacetat auf. Man trennt die wässrige Phase ab und extrahiert die organische Phase erneut zwei mal mit je 50 ml wässriger Natriumhydrogencarbonat-Lösung. Die wässrigen Phasen werden vereinigt und erneut mit 10 ml Ethylacetat exrahiert. Die organischen Phasen werden vereinigt, 2mal mit je 50 ml 1M wässrigem MgSO₄ extrahiert und anschliessend über 2g trokkenem Natriumsulfat getrocknet. Nach dem Abfiltrieren des Salzes wird das Fitrat im Vakuum zur Trockne eingeengt wobei man 320 mg Rohprodukt erhält. Man nimmt den Feststoff in 5ml CH-Cl₃/Methanol(9:1) auf und chromatographiert über einer Kieselgelsäule. Die Produktfraktionen werden vereinigt und am Hochvakuum getrocknet. Das Produkt 5'-Dimethoxytrityl-N⁶-pivaloyl-2'(-1-(2-fluorophenyl)-4-methoxy-piperid-4-yl)-adenosin fällt als ein gelbes Öl in einer Ausbeute von 233 mg (58 % d. Th.) an. ¹H-NMR: (CDCl₃, Standard = TMS, d[ppm]): 8,69: s, 1H, Adenin; 8,48: s, 1H, Adenin; 8,2: s, 1H, Adenin; 7,5-7,2: m, 9H, DMTr; 7,12-6,88: m, 4H, 2-Fluorophenyl; 6,83: 2m, 4H, DMTr; 6,25: d, 1H, 1'; 5,35; m; 4,43: m, 1H; 4,3; m, 1H; 3,76: s, 6H; 3,5, 3,38: 2m, 2H, 5'; 3,15: m, 1H, Fpmp; 2,96: m, 2H; Fpmp; 2,78: s, 3H, CH₃ von Fpmp, m, 1H, FPMP; 2,15-1,75: m, 4H, Fpmp; 1,4: 9H: Piv.

### Beispiel 10: Darstellung von 5'-Dimethoxytrityl-2'(-1-(2-fluorophenyl)-4-methoxy-piperid-4-yl)-uridin aus dem Hydrolysat des 5'-Dimethoxytrityl-2'(-1-(2-fluorophenyl)-4-methoxy-piperid-4-yl)-uridin-3'-yl-cyanoetylphosphorodiisopropylamidits; Umesterung mit Methanol und KF, I₂/H₂O als Quenchlösung ohne I₂-Fänger bei der Aufarbeitung.

In 50 ml einer 0,1M methanolischen KF-Lösung lässt man 10 ml einer Lösung von 0,138 mmol 5'-Dimethoxytrityl-2'(-1-(2-fluorophenyl)-4-methoxy-piperid-4-yl)-uridin-3'-yl-cyanoethyl-H-phosphonat und 0,694mmol Tetrazol in Acetonitrileinfliessen. Die Reaktion ist innerhalb 2 Tagen bei 20-25°C beendet. Die Reaktionsmischung wird mit 5ml Wasser versetzt. Anschliessend wird eine so grosse Menge einer Lösung aus 30 g Jod, 20 ml Wasser, 200ml Pyridin und 750 ml Tetrahydrofuran der Reaktionsmischung zugegeben, bis die braune Jodfarbe für mindestens 5 Minuten bestehen bleibt. Die Reaktionsmischung wird bis zu einem gelblichen Feststoff im Vakuum eingedampft. Man nimmt in einer Emulsion aus 50 ml Wasser und 50 ml Ethylacetat auf. Man trennt die wässrige Phase ab und extrahiert die organische Phase erneut vier mal mit je 50 ml Wasser. Die wässrigen Phasen werden vereinigt und erneut mit 10 ml Ethylacetat extrahiert. Die organischen Phasen werden vereinigt, mit je 50 ml gesättigter NaCl-Lösung extrahiert und anschliessend über 2g trockenem Natriumsulfat getrocknet. Nach dem Abfiltrieren des Salzes wird das Filtrat im Vakuum zur Trockne eingeengt. Man nimmt den Feststoff in 5-10 ml Ethylacetat auf und lässt die so erhaltene Lösung in 200 ml n-Pentan langsam einfliessen. Das Produkt fällt hierbei als weisser amorpher Niederschlag an. Dieser Niederschlag wird abfiltriert und anschliessend mit 50 ml n-Pentan gewaschen. Nach dem Trockenen über P₄O₁₀/KOH für 24 Stunden im Vakuum erhält man 67,7 mg (69 % d. T.) der Verbindung 5'-Dimethoxytrityl-2'-(-1-(2-fluorophenyl)-4-methoxy-piperid-4 -yl)-uridin (75 % d. Th.) an.
¹H-NMR; (CDCl₃, Standard = TMS, d[ppm]); 7,75; d, 1H, H⁶; 7,45-7,15; m, 9H, DMTr; 7,1-6,75: m, 4H, Fpmp, 2m, 4H, DMTr; 6,15: d, 1H, 1'; 5,2: d, 1H, H⁵; 4,74: m, 1H, 2'; 4,33: m, 1H, 3'; 4,15: m, 1H, 4'; 3,76: s, 6H, DMTr; 3,38: m, 2H, 5'; 3,12: s, 3H, CH₃ von Fpmp; 3,2-2,8: m, 4H, Fpmp; 2,15-1,8: m, 4H.

### Beispiel 11: Darstellung von 5'-Dimethoxytrityl-N⁴-benzoyl-2'-(1-(2-fluorophenyl)-4-methoxy-piperid-4-yl)-cytidin aus dem Hydrolysat des 5'-Dimethoxytrityl-N⁴-benzoyl-2'-(1 -(2-fluorophenyl)-4-methoxy-piperid-4-yl)-cytidin-3'-yl-cyanoetylphosphorodiisopropylamidits

In 50 ml eine 0,1 M methanolische KF-Lösung lässt man 10 ml einer Lösung von 0,129 mmol 5'-Dimethoxytrityl-N⁴-benzoyl-2'-(1-(2-fluorophenyl)-4-methoxy-piperid-4-yl)-cytidin-3'-yl-cyanoet hyl-H-phosphonat und 0,645 mmol Tetrazol in Acetonitril einfliessen. Die Reaktion ist innerhalb 2 Tagen bei 20-25°C beendet. Die Reaktionsmischung wird mit 5ml Wasser versetzt. Anschliessend wird eine so grosse Menge einer Lösung aus 30 g Jod, 20 ml Wasser, 200ml pyridin und 750 ml Tetrahydrofuran der Reaktionsmischung zugegeben, bis die braune Jodfarbe für mindestens 5 Minuten bestehen bleibt. Die Reaktionsmischung wird zu einem gelblichen Feststoff im Vakuum eingedamft. Man nimmt in einer Emulsion aus 50 ml Wasser und 50 ml Ethylacetat auf. Man trennt die wässrige Phase ab und extrahiert die organische Phase erneut vier mal mit je 50 ml Wasser. Die wässrigen Phasen werden vereinigt und erneut mit 10 ml Ethylacetat etrahiert. Die organischen Phasen werden vereinigt, mit je 50 ml gesättigter NaCl-Lösung extrahiert und anschliessend über 2g trockenem Natriumsulfat getrocknet. Nach dem Abfiltrieren des Salzes wird das Fitrat im Vakuum zur Trockne eingeengt. Man nimmt den Feststoff in 5-10 ml Ethylacetat auf und lässt die so erhaltene Lösung in 200 ml n-Pentan langsam einfliessen. Das Produkt fällt hierbei als weisser amorpher Niederschlag an. Dieser Niederschlag wird abfiltriert und anschliessend mit 50 ml n-Pentan gewaschen. Nach dem Trockenen über P₄O₁₀/KOH für 24 Stunden im Vakuum erhält man 83 mg (69 % d. T.) der Verbindung 5'-Dimethoxytrityl-N⁴-benzoyl-2'-( 1-(2-fluorophenyl)-4-methoxy-piperid-4-yl)-cytidin (80 % d. Th.) an.
¹H-NMR: (CDCl₃, Standard = TMS, d[ppm]): 8,28: d, 1H; 7,82: d, 2H, Bz; 7,6-7,23: m, 14H, Bz, DMTr, C; 7,03-6,77: d, 4H, DMTr, 2d, 4H, Fpmp; 4,66: t, 1H, 1'; 4,38: t, 1H, 2'; 4,17: m, 1H, 3', 3,74: s, 6H, DMTr; 3,48: m, 2H, 5'; 3,25-2,85: s, 3H, CH₃, Fpmp, m, 4H, Fpmp; 2,18-1,95: m, 4H, Fpmp.¹⁹F-NMR: (CDCl₃, Standard = TFA, d[ppm]): - 123,5.

### Beispiel 12: Darstellung von 5'-Dimethoxytrityl-N⁴-(4-tert.-butylphenoxyacetyl)-2'-deoxycytidin aus dem Hydrolysat des 5'-Dimethoxytrityl-N⁴-(4-tert.-butylphenoxyacetyl)2'-deoxycytidin-3'-yl-cyanoethyl-phosphorodiisopropylamidits.

In 40 ml eine 0,2M methanolische KF Lösung lässt man 4 ml einer acetonitrilischen Lösung, die 200 mg 5'-Dimethoxytrityl-N⁴-(4-tert.-butylphenoxyacetyl)-2'-deoxycy tidin aus dem Hydrolysat des 5'-Dimethoxytrityl-N⁴-(4-tert.-butylphenoxyacetyl)-2'-deoxycytidin-3'-yl-cyanoethyl-H-phosphonat und 2 mmol Tetrazol enthalten einfliessen. Die Dephosphitylierung ist binnen 4 Stunden bei 20-25°C beendet. Die Reaktionsmischung wird nun mit 1 ml Pyridin, dann mit 50 mg Schwefel versetzt. Nach 60 minütigem Rühren bei 20-25°C wird die Reaktionsmischung filtriert und dann auf 100 ml Eiswasser gegossen. Man extrahiert die hieraus resultierende Emulsion drei mal mit je 30 ml Ethylacetat. Die organischen Phasen werden vereinigt, mit je 50 ml gesättigter NaCl-Lösung extrahiert und anschliessend über 2g trockenem Natriumsulfat getrocknet Nach dem Abfiltrieren des Salzes wird das Fitrat im Vakuum zur Trockne eingeengt. Man nimmt den Feststoff in 10 ml Ethylacetat auf und lässt die so erhaltene Lösung in 300 ml n-Hexan langsam einfliessen. Das Produkt fällt hierbei als weisser amorpher Niederschlag an. Dieser Niederschlag wird abfiltriert und anschliessend mit 50 ml n-Pentan gewaschen. Nach dem Trockenen über P₄O₁₀/KOH für 24 Stunden im Vakuum erhält man 112,8 mg (72,1 % d. Th.) der Verbindung 5'-Dimethoxytrityl-N⁴-(4-tert.-butylphenoxy-acetyl)-2'-deoxycytidin.

### Beispiel 13: Umesterung mit Imidazol, Quenchmittel mit I₂/Wasser, Aufarbeitung mit Citratpuffer: Darstellung von 5'-Dimethoxytrityl-(3-N-trifluoroacetylaminopropyl)-2'-deoxyuridin aus der Abflusslösung eines Oligonucleotidsynthesereaktors.

In 80 ml einer Lösung aus 1 g Imidazol pro 50 ml Methanol laufen 5 ml einer im Verlauf der DNA-Synthese anfallenden Abflusslösung des Reaktors ein, die 120 mg 5'-Dimethoxytrityl-(3-N-trifluoroacetylaminopropyl)-2'-deoxyuridin-3'-yl-cyanoethyl-H-phosphonat enthält. Man lässt die Mischung drei Tage bei 20-25°C reagieren. Die Reaktionsmischung wird nun mit 8 ml Wasser versetzt. Anschliessend wird eine so grosse Menge einer Lösung aus 30 g Jod, 20 ml Wasser, 200ml Pyridin und 750 ml Tetrahydrofuran der Reaktionsmischung zugegeben, bis die braune Jodfarbe für mindestens 5 Minuten bestehen bleibt. Die Reaktionsmischung wird zu einem gelblichen Feststoff im Vakuum eingedamft. Man nimmt in einer Emulsion aus 50 ml pH:3-4 Puffer und 40 ml Ethylacetat auf. Der hierbei verwendete Puffer besteht aus 24g NaOH, 117g Zitronensäure und 35g Natriumchlorid pro Liter wässriger Lösung. Man trennt die Imidazol enthaltende wässrige Phase ab und extrahiert die organische Phase erneut zwei mal mit je 50 ml der Pufferlösung. Anschliessend folgt eine Extraktion der organischen Phase mit gesättigter wässriger Natriumhydrogencarbonat Lösung und danach mit 50 ml gesättigter Kochsalz-Lösung. Die organische Phase wird anschliessend über 2g trockenem Natriumsulfat getrocknet. Nach dem Abfiltrieren des Salzes wird das Filtrat im Vakuum zur Trockne eingeengt. Man nimmt den Feststoff in 5-10 ml Ethylacetat auf und lässt die so erhaltene Lösung in 250 ml n-Pentan langsam einfliessen. Das Produkt fällt hierbei als weisser amorpher Niederschlag an. Dieser Niederschlag wird abfiltriert und anschliessend mit 50 ml n-Pentan gewaschen. Nach dem Trockenen über P₄O₁₀/KOH für 24 Stunden im Vakuum erhält man 80 mg (86% d. Th.) der Verbindung 5'-Dimethoxytrityl-(3-Ntrifluoroacetylaminopropyl)-2'-deoxyuridin.
¹H-NMR(CDCl₃, Standard = TMS, d[ppm]): 7,45-6,9: m, 9H, DMTr; 6,73: 2m, 4H, DMTr; 6,48: t, 1H, 1'; 4,56: m, 1H, 3'; 4,04: m, 1H, 4'; 3,7: s, 6H, DMTr; 3,5-3,4: 2m, 3,35-3,25: 2m, 2H, 5'; 2,97: s, 2H, NH₂; 2,47-2,37 m, 1H, 2'; 2,35-2,2 m, 1H, 2'; 1,82, m, 1H; 1,6,m, 1H; 1,45-1,15, m, 4H, Aminopropyl.

### Beispiel 14: Darstellung von 5'-Dimethoxytrityl-(propin-1-yl)-2'-deoxyuridin aus der Abflusslösung eines Oligonucleotidsynthesereaktors.

In 67 ml einer Lösung aus 1 g Imidazol pro 50 ml Methanol laufen 5 ml einer im Verlauf der DNA-Synthese anfallenden Abflusslösung des Reaktors ein, die 250 mg 5'-Dimethoxytrityl-(propin-1-yl)-2'-deoxyuridin-2'-deoxyuridin-3'-yl-cyanoethyl-H-phosphonat enthält. Man lässt drei Tage bei 20-25°C reagieren. Die Reaktionsmischung wird nun mit 8 ml Wasser versetzt. Anschliessend wird eine so grosse Menge einer Lösung aus 30 g Jod, 20 ml Wasser, 200ml Pyridin und 750 ml Tetrahydrofuran der Reaktionsmischung zugegeben, bis die braune Jodfarbe für mindestens 5 Minuten bestehen bleibt.Die Reaktionsmischung wird zu einem gelblichen Feststoff im Vakuum eingedamft. Man nimmt in einer Emulsion aus 50 ml pH:3-4 Puffer und 35 ml Ethylacetat auf. Der hierbei verwendete Puffer besteht aus 24g NaOH, 117g Zitronensäure und 35g Natriumchlorid pro Liter wässriger Lösung. Man trennt die Imidazol enthaltende wässrige Phase ab und extrahiert die organische Phase erneut zwei mal mit je 50 ml der Pufferlösung. Anschliessend folgt eine Extraktion der organischen Phase mit gesättigter wässriger Natriumhydrogencarbonat Lösung und danach mit 50 ml gesättigter Kochsalz Lösung. Die organische Phase wird anschliessend über 2g trockenem Natriumsulfat getrocknet. Nach dem Abfiltrieren des Salzes wird das Filtrat im Vakuum zur Trockne eingeengt. Man nimmt den Feststoff in 5-10 ml Ethylacetat auf und lässt die so erhaltene Lösung in 250 ml n-Pentan langsam einfliessen. Das Produkt fällt hierbei als weisser amorpher Niederschlag an. Dieser Niederschlag wird abfiltriert und anschliessend mit 50 ml n-Pentan gewaschen. Nach dem Trockenen über P₄O₁₀/KOH für 24 Stunden im Vakuum erhält man 125 mg (68 % d. Th.) der Verbindung 5'-Dimethoxytrityl-(propin-1-yl)-2'-deoxyuridin.
¹H-NMR(CDCl₃, Standard = TMS, d[ppm]): 7,95: s, 1H, NH; 7,4-7,15: m, 10H, DMTr; 6,78: 2s, 4H, DMTr; 6,24: t, 1H, 1'; 4,5: m, 3'; 4,06: m, 4'; 3,73: s, 6H, DMTr, 3,28: m, 2H, 5'; 2,43: m, 1H, 2'; 2,22: m, 1H, 2'; 1,61: s, 3H, CH₃ von propinyl.

### Beispiel 15: Darstellung von 5'-Dimethoxytrityl-(3-trifluoroaminopropin-1-yl)-2'-deoxyuridin aus der Abflusslösung eines Oligonucleotidsynthesereaktors.

In 78 ml einer Lösung aus 1 g Imidazol pro 50 ml Methanol laufen 5 ml einer im Verlauf der DNA-Synthese anfallenden Abflusslösung des Reaktors ein, die 200 mg 5'-Dimethoxytrityl-(3-aminopropin-1-yl)-2'-deoxyuridin-2'-deoxyuridin-3'-yl-cyanoethyl-H-phosphonat enthalten. Man lässt drei Tage bei 20-25°C reagieren. Die Reaktionsmischung wird nun mit 7,8 ml Wasser versetzt. Anschliessend wird eine so grosse Menge einer Lösung aus 30 g Jod, 20 ml Wasser, 200ml Pyridin und 750 ml Tetrahydrofuran der Reaktionsmischung zugegeben, bis die braune Jodfarbe für mindestens 5 Minuten bestehen bleibt. Die Reaktionsmischung wird zu einem gelblichen Feststoff im Vakuum eingedamft. Man nimmt ihn in einer Emulsion aus 50 ml pH-3-4 Puffer und 40 ml Ethylacetat auf. Der hierbei verwendete Puffer besteht aus 24g NaOH, 117g Zitronensäure und 35g Natriumchlorid pro Liter wässriger Lösung. Man trennt die Imidazol enthaltende wässrige Phase ab und extrahiert die organische Phase erneut zwei mal mit je 50 ml der Pufferlösung. Anschliessend folgt eine Extraktion der organischen Phase mit gesättigter wässriger Natriumhydrogencarbonat Lösung und danach mit 50 ml gesättigter Kochsalz Lösung. Die organische Phase wird anschliessend über 2g trockenem Natriumsulfat getrocknet. Nach dem Abfiltrieren des Salzes wird das Fitrat im Vakuum zur Trockne eingeengt. Man nimmt den Feststoff in 5-10 ml Ethylacetat auf und lässt die so erhaltene Lösung in 250 ml n-Pentan langsam einfliessen. Das Produkt fällt hierbei als weisser amorpher Niederschlag an. Dieser Niederschlag wird abfiltriert und anschliessend mit 50 ml n-Pentan gewaschen. Nach dem Trockenen über P₄O₁₀/KOH für 24 Stunden im Vakuum erhält man 113 mg (73 % d. Th.) der Verbindung 5'-Dimethoxytrityl-(3-trifluoroacetylaminopropin1-yl)-2'-deoxyuridin.
¹H-NMR(CDCl₃, Standard = TMS, d[ppm]): 8,15: s, 1H, NH; 7,4-7,15: 10H, DMTr, H⁶; 6,26: t, 1H, 1'; 4,53: m, 1H, 3'; 3,97: m, 2H, 5';3,73; s, 6H, DMTr; 3,3; m, 2H, CH₂ von Propinyl; 2,48, m, 1H, 2'; 2,25: m, 1H, 2'.

### Beispiel 16: Darstellung von N-5'-Dimethoxytrityl-3'-deoxythymidin-3'-yl,N-propyl-3'-deoxythymid-5'-yl)-acetamid aus der Abflusslösung eines Oligonucleotid-synthesereaktors.

In 78 ml einer Lösung aus 1 g Imidazol pro 50 ml Methanol laufen 5 ml einer im Verlauf der DNA-Synthese anfallenden Abflusslösung des Reaktors ein, die 200 mg N-5'-Dimethoxytrityl-3'-deoxythymidin-3'-yl,N-propyl-3'-deoxythymid-3'-cyanoethyl-H-phosphonyl-5'-yl)-acetamid enthält. Man lässt sieben Tage bei 20-25°C reagieren. Die Reaktionsmischung wird nun mit 20 ml Wasser versetzt. Anschliessend wird eine so grosse Menge einer Lösung aus 30 g Jod, 20 ml Wasser, 200 ml Pyridin und 750 ml Tetrahydrofuran der Reaktionsmischung zugegeben, bis die braune Jodfarbe für mindestens 5 Minuten bestehen bleibt. Die Reaktionsmischung wird zu einem gelblichen Feststoff im Vakuum eingedamft. Man nimmt ihn in einer Emulsion aus 50 ml pH-3-4 Puffer und 40 ml Ethylacetat auf. Der hierbei verwendete Puffer besteht aus 24g NaOH, 117g Zitronensäure und 35g Natriumchlorid pro Liter wässriger Lösung. Man trennt die Imidazol enthaltende wässrige Phase ab und extrahiert die organische Phase erneut zwei mal mit je 50 ml der Pufferlösung sukzessive. Anschliessend folgt eine Extraktion der organischen Phase mit gesättigter wässriger Natriumhydrogencarbonat Lösung und danach mit 50 ml gesättigter Kochsalz Lösung. Die organische Phase wird anschliessend über 2g trockenem Natriumsulfat getrocknet. Nach dem Abfiltrieren des Salzes wird das Fitrat im Vakuum zur Trockne eingeengt. Man nimmt den Feststoff in 3 ml Ethylacetat/CHCl₃ (1:1, V:V) auf und lässt die so erhaltene Lösung in 600 ml n-Pentan langsam einfliessen. Das Produkt fällt hierbei als weisser amorpher Niederschlag an. Dieser Niederschlag wird abfiltriert und anschliessend mit 50 ml n-Pentan gewaschen. Nach dem Trockenen über P₄O₁₀/KOH für 24 Stunden im Vakuum erhält man 107 mg (66% d. Th.) der Verbindung N-5'-Dimethoxytrityl-3'-deoxythymidin-3'-yl,N-propyl-3'-deoxthymid-5'-yl)-acetamid.
¹H-NMR(CDCl₃, Standard = TMS, d[ppm]): ¹H-NMR(CDCl₃, Standard = TMS, d[ppm]): 7,65: s, 1H, H⁶; 7,4-7,15: m, 9H, DMTr; 7,1: s, 1H, H⁶; 6,77: 2s, 4H, DMTr; 6,53: t, 1H, 1'; 6,13: t, 1H, 1'; 4,28: m, 1H, 3'; 4,1: m, 2H, 3',4'; 3,72: s, 6H, DMTr; m, 1H; 3,52: m, 4', 1H; 3,5, d, 1H; 3,15-2,85: m, 3H, 5', CH₂ von acetamid; 2,56; m, 1H, 2'; 2,45-2,25: m, 3H, 2' CH₂ von Propyl; 2,2-2,1: 2H, 2'; 1,83: s, 3H, CH₃⁵; 1,45: s, 3H,CH₃⁵; 1,22: m, 2H, CH₂ von Propyl; 0,73: t, 3H, CH₃ von Propyl.

### Beispiel 17: Darstellung von 5'-Dimethoxytrityl-3'-propyl-5-methyluridin aus der Abflusslösung eines Oligonucleotidsynthesereaktors.

In 100 ml einer Lösung aus 1 g Imidazol pro 50 ml Methanol laufen 5 ml einer im Verlauf der DNA-Synthese anfallenden Abflusslösung des Reaktors ein, die 400 mg 5'-Dimethoxytrityl-3'-propyl-5-methyluridin-3'-cyanoethyl-H -phosphonat enthält. Man lässt sieben Tage bei 20-25°C reagieren. Die Reaktionsmischung wird nun mit 20 ml Wasser versetzt. Anschliessend wird eine so grosse Menge einer Lösung aus 30 g Jod, 20 ml Wasser, 200 ml Pyridin und 750 ml Tetrahydrofuran der Reaktionsmischung zugegeben, bis die braune Jodfarbe für mindestens 5 Minuten bestehen bleibt. Die Reaktionsmischung wird zu einem gelblichen Feststoff im Vakuum eingedamft. Man nimmt in einer Emulsion aus 50 ml pH-3-4 Puffer und 40 ml Ethylacetat auf. Der hierbei verwendete Puffer besteht aus 24g NaOH, 117g Zitronensäure und 35g Natriumchlorid pro Liter wässriger Lösung. Man trennt die Imidazol enthaltende wässrige Phase ab und extrahiert die organische Phase erneut zwei mal mit je 50 ml der Pufferlösung. Anschliessend folgt eine Extraktion der organischen Phase mit gesättigter wässriger Natriumhydrogencarbonat Lösung und danach mit 50 ml gesättigter Kochsalz Lösung. Die organische Phase wird anschliessend über 2g trockenem Natriumsulfat getrocknet. Nach dem Abfiltrieren des Salzes wird das Fitrat im Vakuum zur Trockne eingeengt. Nach dem Trockenen über P₄O₁₀/KOH für 24 Stunden im Vakuum erhält man 258 mg(86% d. Th.) der Verbindung 5'-Dimethoxytrityl-3'-propyl-5-methyluridin als gelbes Öl.
¹H-NMR(CDCl₃, Standard = TMS, d[ppm]); 8,45: s, 1H, NH; 7,57: s, 1H, H⁶; 7,45-7,15: m, 9H, DMTr; 6,75: 2s, 4H, DMTr; 5,9: d, 1H, 1'; 4,38; m, 1H, 3'; 4,0: m, 1H, 4'; 3,95: m, 1H, 2'; 3,72: s, 6H, DMTr,; 3,8-3,4: m, 2H: propyl; 3,6-3,3: 4m, 2H, 5'; 1,56: m, 2H, propyl; 1,88: t, 3H, propyl.

### Beispiel 18: Umesterung eines Phosphits auf einem Polymer mittels Methanol als Akzeptoralkohol

685 mg eines mit Thymidin-5'-cyanoethyl-H-phosphonat derivatisierten Controlled Pore Glass (CPG), das eine Belegungdichte des Nucleosids von 36 mmol/g aufweist, wird mit 10 ml einer Lösung aus 2% N-Methylmorpholin in Methanol umgesetzt. Das Nucleosid ist hierbei über eine Succinylgruppe zu seiner 3'-OH-Gruppe mit dem CPG kovalent verbunden. Nach einer Stunde wird eine Probe von 137 mg der festen Phase aus dem Reaktionsgemisch entfernt. Der Träger wird 4 mal mit je 2 ml Acetonitril gewaschen und anschliessend mit einer Lösung aus 30 g Jod, 20 ml Wasser, 200ml Pyridin und 750 ml Tetrahydrofuran eine Stunde umgesetzt. Anschliessend wird die feste Phase 10 mal mit je 2 ml Acetonitril, dann 10 mal mit je 2 ml Diethyletherther gewaschen. Der Träger wird getrocknet und bei 55-60°C mit konzentrierter wässriger Ammoniaklösung behandelt. Anschliessend wird die feste Phase, an der sich nun kein Nukleosid mehr befindet abfiltriert. Das Filtrat wird zunächst lyophilisiert, dann mit 0,1 M Triethylammoniumacetat bei pH 7 aufgenommen und mittels HPLC analysiert. Man erkennt ein Gemisch aus 5'-Thymidinmonophosphat, Thymidin und Thymidin-5'-methylphosphat. Das Thymidin-5'-Phosphat ist aus dem Edukt durch Jodoxidation und Ammonolyse hervorgegangen. Das Thymidin-5'-methylphosphat ist aus einem Zwischenprodukt der Dephosphitylierung ,dem Thymidin-5-methyl-H-phosphonat bei der oxidativen und hydrolytischen Aufarbeitung hervorgegangen. Das Thymidin ist aus dem Produkt der Dephosphitylierung, dem polymer gebundenen Thymidin nach Verseifung der Succinylgruppe-Brückengruppe hervorgegangen. Die Restmenge des Trägers wird noch 36 Stunden der vorgenannten oxidativen und ammonolytischen Aufarbeitung unterzogen. Man erhält ausschliesslich Thymidin in dem Filtrat des Trägers.

### Beispiel 19: Umesterung eines Phosphits auf einem Polymer mittels Cyanoethanol als Akzeptoralkohol

139 mg eines mit Thymidin-5'-cyanoethyl-H-phosphonat derivatisierten Controlled Pore Glass (CPG), das eine Belegungdichte des Nucleosids von 36 mmol/g aufweist, wird mit 10 ml einer Lösung aus 10% N-Methylmorpholin in Cyanoethanol umgesetzt. Das Nukleosid ist hierbei über eine Succinylgruppe zu seiner 3'-OH-Gruppe mit dem CPG kovalent verbunden. Nach 100 Minuten wird der Träger 4 mal mit je 2 ml Acetonitril gewaschen und anschliessend mit einer Lösung aus 30 g Jod, 20 ml Wasser, 200ml Pyridin und 750 ml Tetrahydrofuran eine Stunde umgesetzt. Anschliessend wird die feste Phase 10 mal mit je 2 ml Acetonitril, dann 10 mal mit je 2 ml Diethylether gewaschen. Der Träger wird getrocknet und bei 55-60°C mit konzentrierte wässriger Ammoniaklösung behandelt. Anschliessend wird die feste Phase, an der sich nun kein Nukleosid mehr befindet abfiltriert. Das Filtrat wird zunächst lyophilisiert, dann mit 0,1 M Triethylammoniumacetat pH: 7 aufgenommen und mittels HPLC analysiert. Man erkennt ein Gemisch aus 5'-Thymidinmonophosphat und Thymidin im Verhältnis 3:97. Das Thymidin-5'-Phosphat ist aus dem Edukt durch Jodoxidation und Ammonolyse hervorgegangen. Das Thymidin ist aus dem Produkt der Dephosphitylierung, dem polymer gebundenen Thymidin nach Verseifung der Succinylgruppe-Brückengruppe, hervorgegangen.

### Beispiel 20: Umesterung eines Phosphits auf einem Polymer mittels Methanol als Akzeptoralkohol in Anwesenheit von Magnesium-di-trifluoracetat Mg(OTf)₂.

139 mg eines mit Thymidin-5'-cyanoethyl-H-phosphonat derivatisierten Controlled Pore Glass (CPG), das eine Belegungdichte des Nukleosids von 36 mmol/g aufweist, wird mit 10 ml einer Lösung aus 0,1 M Mg(OTf)₂ in N-Methylimidazol : Methanol (1:9) umgesetzt. Das Nukleosid ist hierbei über eine Succinylgruppe zu seiner 3'-OH-Gruppe mit dem CPG kovalent gebunden. Nach 30 Minuten wird der Träger 4 mal mit je 2 ml Acetonitril gewaschen und anschliessend mit einer Lösung aus 30 g Jod, 20 ml Wasser, 200ml Pyridin und 750 ml Tetrahydrofuran eine Stunde umgesetzt. Anschliessend wird die feste Phase 10 mal mit je 2 ml Acetonitril, dann 10 mal mit je 2 ml Ether gewaschen, Der Träger wird getrocknet und bei 55-60°C mit konzentrierte wässriger Ammoniaklösung behandelt. Anschliessend wird die feste Phase, an der sich nun kein Nukleosid mehr befindet abfiltriert. Das Filtrat wird zunächst lyophilisiert, dann mit 0,1 M Triethylammoniumacetat pH: 7 aufgenommen und mittels HPLC analysiert. Man erkennt ein Gemisch aus Thymidin und Thymidin-5'-methylphosphat. Das Thymidin -5'-methylphosphat ist aus einem Zwischenprodukt der Dephosphitylierung,dem Thymidin-5-methyl-H-phosphonat bei der oxidativen und hydrolyrischen Aufarbeitung hervorgegangen. Das Thymidin, ist aus dem Produkt der Dephosphitylierung, des polymer gebundenen Thymidin nach Verseifung der Succinylgruppe-Brückengruppe. Das Verhältnis Thymidin zu Thymidin-5'-monophosphat ist 97:3.

### Beispiel 21: Umesterung eines Phosphits auf einemPolymer mittels Cyanoethanol als Akzeptoralkohol in Anwesenheit von LiCl.

139 mg eines mit Thymidin-5'-cyanoethyl-H-phosphonat derivatisierten Controlled Pore Glass (CPG), das eine Belegungdichte des Nukleosids von 36 mmol/g aufweist, wird mit 10 ml einer Lösung aus 0,1 M LiCl in N-Methylmorpholin: Cyanoethanol (1:9) umgesetzt. Das Nukleosid ist hierbei über eine Succinylgruppe zu seiner 3'-OH-Gruppe mit dem CPG kovalent verbunden. Nach 30 Minuten wird der Träger 4 mal mit je 2 ml Acetonitril gewaschen und anschliessend mit einer Lösung aus 30 g Jod, 20 ml Wasser, 200ml Pyridin und 750 ml Tetrahydrofuran eine Stunde umgesetzt. Anschliessend wird die feste Phase 10 mal mit je 2 ml Acetonitril, dann 10 mal mit je 2 ml Diethylether gewaschen. Der Träger wird getrocknet und bei 55-60°C mit konzentrierter wässriger Ammoniaklösung behandelt. Anschliessend wird die feste Phase, an der sich nun kein Nukleosid mehr befindet, abfiltriert. Das Filtrat wird zunächst lyophilisiert, dann mit 0,1 M Triethylammoniumacetat pH: 7 aufgenommen und mittels HPLC analysiert. Man erkennt ein Gemisch aus Thymidin und Thymidin-5'-phosphat. Das Thymidin-5'-Phosphat ist aus dem Edukt durch Jodoxidation und Ammonolyse hervorgegangen. Das Thymidin ist aus dem Produkt der Dephosphitylierung des polymer gebundenen Thymidin nach Verseifung der Succinylgruppe-Brückengruppe hervorgegangen. Das Verhältnis Thymidin zu Thymidin-5'-monophosphat ist 98:2.

## Patentansprüche

1. Verfahren zur Herstellung von natürlichen oder synthetischen Nukleosiden, Nukleosidanalogen oder Oligonukleotiden aus mindestens zwei solcher Nukleoside und/oder Nukleosidanalogen, deren Grundgerüst einen gegebenenfalls substituierten Rest einer Nukleinbase B und eine geschützte Hydroxylgruppe der Formel R₁-O-, worin R₁ für eine Schutzgruppe steht, enthält, oder die mit dem Sauerstoffatom einer Hydroxylgruppe direkt oder über eine Brückengruppe an ein festes Trägermaterial gebunden sind, aus Nukleosidmonomeren oder aus Oligonukleotiden, an deren Grundgerüst neben besagten Resten eine Gruppe der Formeln I, Ia oder Ib 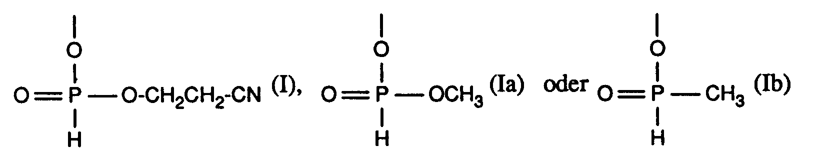 gebunden ist, durch Überführung der Gruppe der Formeln I, Ia oder Ib in eine Hydroxylgruppe -OH, indem man die Nukleoside oder Oligonukleotide, gegebenenfalls in einem inerten organischen Lösungsmittel, mit einem Überschuss eines aliphatischen, cycloaliphatischen, araliphatischen oder aromatischen Alkohols mit 1 bis 30 C-Atomen, einem Polyol mit 2 bis 50 C-Atomen oder einem polymeren Polyol in Gegenwart einer mindestens katalytischen Menge einer anorganischen Base oder einer organischen Stickstoffbase umsetzt, wobei die Base einen pK-Wert von 4 bis 10 aufweist.

2. Verfahren gemäss Anspruch 1, dadurch gekennzeichnet, dass das Lösungsmittel ausgewählt ist aus der Gruppe bestehend aus Ethern, halogenierten Kohlenwasserstoffen, Carbonsäureamiden, Lactamen, Sulfoxiden, Sulfonen, aromatischen Kohlenwasserstoffen, Nitrilen, aliphatischen und cycloaliphatischen Kohlenwasserstoffen.

3. Verfahren gemäss Anspruch 2, dadurch gekennzeichnet, dass das Lösungsmittel ausgewählt ist aus der Gruppe bestehend aus Ethern, halogenierten Kohlenwasserstoffen, Nitrilen, Carbonsäureamiden und Lactamen.

4. Verfahren gemäss Anspruch 3, dadurch gekennzeichnet, dass das Lösungsmittel Tetrahydrofuran oder Dioxan ist.

5. Verfahren gemäss Anspruch 3, dadurch gekennzeichnet, dass das Lösungsmittel ausgewählt ist aus der Gruppe bestehend aus Acetonitril, Propionitril, Benzonitril und Phenylacetonitril.

6. Verfahren gemäss Anspruch 1, dadurch gekennzeichnet, dass der aliphatische, cycloaliphatische, araliphatische oder aromatische Alkohol, das Polyol oder das polymere Polyol in einem Überschuss von mindestens 100 Moläquivalenten und bis zu 1000 Moläquivalenten Hydroxylgruppen pro Moläquivalent Gruppen der Formel I vorliegt.

7. Verfahren gemäss Anspruch 1, dadurch gekennzeichnet, dass die Umsetzung bei einer Temperatur von -20°C bis 55°C erfolgt.

8. Verfahren gemäss Anspruch 7, dadurch gekennzeichnet, dass die Umsetzung bei einer Temperatur von 15°C bis 35°C erfolgt.

9. Verfahren gemäss Anspruch 8, dadurch gekennzeichnet, dass die Umsetzung bei Raumtemperatur erfolgt.

10. Verfahren gemäss Anspruch 1, dadurch gekennzeichnet, dass der Alkohol ein Alkanol mit 1 bis 20 Kohlenstoffatomen ist.

11. Verfahren gemäss Anspruch 10, dadurch gekennzeichnet, dass der Alkohol ein Alkanol mit 1 bis 12 Kohlenstoffatomen ist.

12. Verfahren gemäss Anspruch 11, dadurch gekennzeichnet, dass der Alkohol ein Alkanol mit 1 bis 6 Kohlenstoffatomen ist.

13. Verfahren gemäss Anspruch 10, dadurch gekennzeichnet, dass der Alkohol ausgewählt ist aus der Gruppe bestehend aus Methanol, Ethanol, n- und i-Propanol, n-, i- und t-Butanol, Pentanol, Hexanol, Ethylenglykol-, Diethylenglykolmonomethyl- und - monoethylether.

14. Verfahren gemäss Anspruch 11, dadurch gekennzeichnet, dass der Alkohol ein Alkanol mit 1 bis 4 Kohlenstoffatomen ist.

15. Verfahren gemäss Anspruch 14, dadurch gekennzeichnet, dass der Alkohol Methanol ist.

16. Verfahren gemäss Anspruch 1, dadurch gekennzeichnet, dass der Alkohol ein Cycloalkanol mit 5 bis 12 Kohlenstoffatomen ist.

17. Verfahren gemäss Anspruch 16, dadurch gekennzeichnet, dass der Alkohol ein Cycloalkanol mit 5 bis 8 Kohlenstoffatomen ist.

18. Verfahren gemäss Anspruch 16, dadurch gekennzeichnet, dass der Alkohol ausgewählt ist aus der Gruppe bestehend aus Cyclopentanol, Cyclohexanol, Methylcyclohexanol, Cycloheptanol und Cyclooctanol.

19. Verfahren gemäss Anspruch 1, dadurch gekennzeichnet, dass der Alkohol ein araliphatischer Alkohol mit 7 bis 30 Kohlenstoffatomen ist.

20. Verfahren gemäss Anspruch 19, dadurch gekennzeichnet, dass der Alkohol ein araliphatischer Alkohol mit 7 bis 20 Kohlenstoffatomen ist.

21. Verfahren gemäss Anspruch 19, dadurch gekennzeichnet, dass der Alkohol ausgewählt ist aus der Gruppe bestehend aus un-, Alkyl- oder Alkoxy-substituiertem Benzylalkohol und β-Phenylethanol.

22. Verfahren gemäss Anspruch 1, dadurch gekennzeichnet, dass der Alkohol ein aromatischer Alkohol mit 6 bis 30 Kohlenstoffatomen ist.

23. Verfahren gemäss Anspruch 22, dadurch gekennzeichnet, dass der Alkohol ein aromatischer Alkohol mit 6 bis 12 Kohlenstoffatomen ist.

24. Verfahren gemäss Anspruch 22, dadurch gekennzeichnet, dass der Alkohol ausgewählt ist aus der Gruppe bestehend aus Phenol, Naphthol und un-, Alkyl- oder Alkoxy-substituiertem Phenol oder Naphthol.

25. Verfahren gemäss Anspruch 1, dadurch gekennzeichnet, dass das Polyol 2 bis 20 Kohlenstoffatomen enthält.

26. Verfahren gemäss Anspruch 25, dadurch gekennzeichnet, dass das Polyol ausgewählt ist aus der Gruppe bestehend aus Ethylenglykol, Propylenglykol, Butandiol, Pentandiol, Hexandiol, Cyclohexandiol, Dihydromethylcyclohexan, Diethylenglykol, Triethylenglykol, Polyethylenglykol, Trimethylolpropan, Pentaerythrit, Dipentaerythrit, Glycerin und Sacchariden.

27. Verfahren gemäss Anspruch 1, dadurch gekennzeichnet, dass das polymere Polyol ausgewählt ist aus der Gruppe bestehend aus Polyvinylalkohol, Polysaccharide und Polyacryl-bzw. -methacrylsäurehydroxyalkylester.

28. Verfahren gemäss Anspruch 1, dadurch gekennzeichnet, dass die anorganische Base ausgewählt ist aus der Gruppe bestehend aus basischem Metalloxid, Schwermetalloxid und -hydroxid.

29. Verfahren gemäss Anspruch 28, dadurch gekennzeichnet, dass die anorganische Base basisches Aluminiumoxid ist.

30. Verfahren gemäss Anspruch 28, dadurch gekennzeichnet, dass die anorganische Base basisches Aluminiumoxid ist.

31. Verfahren gemäss Anspruch 1, dadurch gekennzeichnet, dass die katalytische Menge Base im Bereich von 0,01 bis 20 Mol % liegt.

32. Verfahren gemäss Anspruch 1, dadurch gekennzeichnet, dass die organische Stickstoffbase ausgewählt ist aus der Gruppe bestehend aus aromatisches Amin, aromatischer N-Heterocyclus und N-Alkylmorpholin.

33. Verfahren gemäss Anspruch 32, dadurch gekennzeichnet, dass die Base ausgewählt ist aus der Gruppe bestehend aus Imidazol, Pyridin, Polyvinylpyridin, N-Methylmorpholin, Anilin und o-Diaminobenzol.

34. Verfahren gemäss Anspruch 1, dadurch gekennzeichnet, dass die Nukleoside und Oligonukleotide an ein festes Trägermaterial gebunden sind.

35. Verfahren gemäss Anspruch 1, dadurch gekennzeichnet, dass zusätzlich katalytische Mengen einer nukleophilen anorganischen Base zugegen sind, die die Umsetzung katalysieren.

36. Verfahren gemäss Anspruch 1, dadurch gekennzeichnet, dass zusätzlich katalytische Mengen einer Lewissäure zugegen sind.

## Claims

1. Process for the preparation of natural or synthetic nucleosides, nucleoside analogues or oligonucleotides from at least two such nucleosides and/or nucleoside analogues, whose basic molecule contains an unsubstituted or substituted radical of a nucleic base B and a protected hydroxyl group of formula R₁-O-₁, wherein R₁ is a protecting group, or which are bonded with the oxygen atom of a hydroxyl group directly or by a bridging group to a solid carrier material, from nucleoside monomers or from oligonucleotides, to whose basic molecule is bonded, in addition to said radicals, a group of formulae I, Ia or Ib 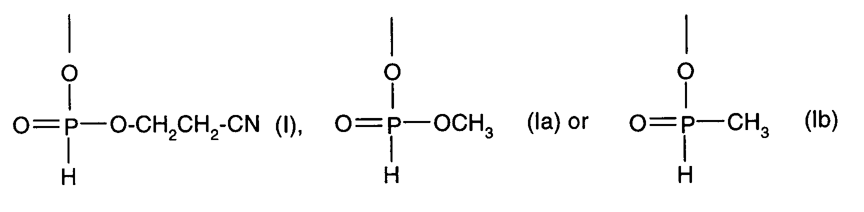 by converting the group of formula I, Ia or Ib into a hydroxyl group -OH by reacting the nucleosides or oligonucleotides, in the absence or presence of an inert organic solvent, with an excess of an aliphatic, cycloaliphatic, araliphatic or aromatic alcohol containing 1 to 30 carbon atoms, a polyol containing 2 to 50 carbon atoms or a polymeric polyol, in the presence of at least a catalytic amount of an inorganic base or of an organic nitrogen base, which base has a pK value of 4 to 10.

2. A process according to claim 1, characterised in that the solvent is selected from the group consisting of ethers, halogenated hydrocarbons, carboxamides, lactams, sulphoxides, sulphones, aromatic hydrocarbons, nitriles, aliphatic and cycloaliphatic hydrocarbons.

3. A process according to claim 2, characterised in that the solvent is selected from the group consisting of ethers, halogenated hydrocarbons, nitriles, carboxamides and lactams.

4. A process according to claim 3, characterised in that the solvent is tetrahydrofuran or dioxane.

5. A process according to claim 3, characterised in that the solvent is selected from the group consisting of acetonitrile, propionitrile, benzonitrile and phenylacetonitrile.

6. A process according to claim 1, characterised in that the aliphatic, cycloaliphatic, araliphatic or aromatic alcohol, the polyol or the polymeric polyol is present in an excess of at least 100 molar equivalents and up to 1000 molar equivalents of hydroxyl groups per molar equivalent of groups of formula 1.

7. A process according to claim 1, characterised in that the reaction is carried out at a temperature of -20°C to 55°C.

8. A process according to claim 7, characterised in that the reaction is carried out at a temperature of 15°C to 35°C.

9. A process according to claim 8, characterised in that the reaction is carried out at room temperature.

10. A process according to claim 1, characterised in that the alcohol is an alkanol with 1 to 20 carbon atoms.

11. A process according to claim 10, characterised in that the alcohol is an alkanol with 1 to 12 carbon atoms.

12. A process according to claim 11, characterised in that the alcohol is an alkanol with 1 to 6 carbon atoms.

13. A process according to claim 10, characterised in that the alcohol is selected from the group consisting of methanol, ethanol, n- and isopropanol, n-, iso- and tert.-butanol, pentanol, hexanol, ethylene glycol and diethylene glycol monomethyl and monoethyl ether.

14. A process according to claim 11, characterised in that the alcohol is an alkanol with 1 to 4 carbon atoms.

15. A process according to claim 14, characterised in that the alcohol is methanol.

16. A process according to claim 1, characterised in that the alcohol is a cycloalkanol with 5 to 12 carbon atoms.

17. A process according to claim 16, characterised in that the alcohol is a cycloalkanol with 5 to 8 carbon atoms.

18. A process according to claim 16, characterised in that the alcohol is selected from the group consisting of cyclopentanol, cyclohexanol, methylcyclohexanol, cycloheptanol and cyclooctanol.

19. A process according to claim 1, characterised in that the alcohol is an araliphatic alcohol with 7 to 30 carbon atoms.

20. A process according to claim 19, characterised in that the alcohol is an araliphatic alcohol with 7 to 20 carbon atoms.

21. A process according to claim 19, characterised in that the alcohol is selected from the group consisting of unsubstituted or alkyl- or alkoxy-substituted benzyl alcohol and ß-phenylethanol.

22. A process according to claim 1, characterised in that the alcohol is an aromatic alcohol with 6 to 30 carbon atoms.

23. A process according to claim 22, characterised in that the alcohol is an aromatic alcohol with 6 to 12 carbon atoms.

24. A process according to claim 22, characterised in that the alcohol is selected from the group consisting of phenol, naphthol and unsubstituted and alkyl- or alkoxy-substituted phenol or naphthol.

25. A process according to claim 1, characterised in that the polyol contains 2 to 20 carbon atoms.

26. A process according to claim 25, characterised in that the polyol is selected from the group consisting of ethylene glycol, propylene glycol, butanediol, pentanediol, hexanediol, cyclohexanediol, dihydromethylcyclohexane, diethylene glycol, triethylene glycol, polyethylene glycol, trimethylolpropane, pentaerythritol, dipentaerythritol, glycerol and saccharides.

27. A process according to claim 1, characterised in that the polymeric polyol is selected from the group consisting of polyvinyl alcohol, polysaccharides and hydroxyalkyl polyacrylates and hydroxyalkyl polymethacrylates.

28. A process according to claim 1, characterised in that the inorganic base is selected from the group consisting of basic metal oxide, heavy metal oxide and heavy metal hydroxide.

29. A process according to claim 28, characterised in that the inorganic base is basic alumina.

30. A process according to claim 28, characterised in that the inorganic base is basic alumina.

31. A process according to claim 1, characterised in that the catalytic amount of base lies in the range 0.01 to 20 % molar.

32. A process according to claim 1, characterised in that the organic nitrogen base is selected from the group consisting of aromatic amine, aromatic N-heterocycle and N-alkylmorpholine.

33. A process according to claim 32, characterised in that the base is selected from the group consisting of imidazole, pyridine, polyvinyl pyridine, N-methylmorpholine, aniline and o-diaminobenzene.

34. A process according to claim 1, characterised in that the nucleosides and oligonucleotides are bound to a solid carrier.

35. A process according to claim 1, characterised in that catalytic amounts of a nucleophilic inorganic base are additionally present, and these catalyse the reaction.

36. A process according to claim 1, characterised in that catalytic amounts of a Lewis acid are additionally present.

## Revendications

1. Procédé de préparation de nucléosides, d'analogues de nucléosides ou d'oligonucléotides, naturels ou synthétiques, à partir d'au moins deux de ces nucléosides et/ou analogues de nucléosides, dont l'ossature fondamentale contient un radical éventuellement substitué d'une base nucléine B et un groupe hydroxyle protégé de formule R₁-O, dans laquelle R₁ représente un groupe protecteur, ou qui sont liés avec l'atome d'oxygène d'un groupe hydroxyle de façon directe ou par l'intermédiaire d'un groupe de pont à un support solide, de monomères de nucléosides ou d'oligonucléotides, à l'ossature fondamentale desquels est lié, outre les radicaux mentionnés, un groupe de formules I, Ia, ou Ib 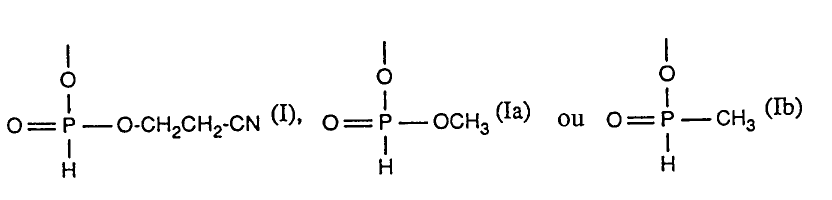 par transformation du groupe de formules I, Ia ou Ib dans un groupe hydroxyle -OH, en faisant réagir les nucléosides ou oligonucléotides, le cas échéant dans un solvant organique inerte, avec un excès d'un alcool aliphatique, cycloaliphatique, araliphatique ou aromatique ayant 1 à 30 atomes de carbone, un polyol ayant 2 à 50 atomes de carbone ou un polyol polymère en présence d'une quantité au moins catalytique d'une base inorganique ou d'une base azotée organique, la base présentant un pK de 4 à 10.

2. Procédé selon la revendication 1, caractérisé en ce que le solvant est choisi dans le groupe constitué par les éthers, les hydrocarbures halogénés, les amides d'acide carboxylique, les lactames, les sulfoxydes, les sulfones, les hydrocarbures aromatiques, les nitriles, les hydrocarbures aliphatiques et cycloaliphatiques.

3. Procédé selon la revendication 2, caractérisé en ce que le solvant est choisi dans le groupe constitué par les éthers, les hydrocarbures halogénés, les nitriles, les amides d'acide carboxylique et les lactames.

4. Procédé selon la revendication 3, caractérisé en ce que le solvant est le tétrahydrofuranne ou le dioxanne.

5. Procédé selon la revendication 3, caractérisé en ce que le solvant est choisi dans le groupe constitué par l'acétonitrile, le propionitrile, le benzonitrile et le phénylacétonitrile.

6. Procédé selon la revendication 1, caractérisé en ce que l'alcool aliphatique, cycloaliphatique, araliphatique ou aromatique, le polyol ou le polyol polymère se présentent dans un excès d'au moins 100 équivalents molaires et jusqu'à 1000 équivalents molaires de groupes hydroxyle par équivalent molaire de groupes de formule I.

7. Procédé selon la revendication 1, caractérisé en ce que la réaction se déroule à une température de -20°C à 55°C.

8. Procédé selon la revendication 7, caractérisé en ce que la réaction se déroule à une température de 15°C à 35°C.

9. Procédé selon la revendication 8, caractérisé en ce que la réaction se déroule à la température ambiante.

10. Procédé selon la revendication 1, caractérisé en ce que l'alcool est un alcanol ayant 1 à 20 atomes de carbone.

11. Procédé selon la revendication 10, caractérisé en ce que l'alcool est un alcanol ayant 1 à 12 atomes de carbone.

12. Procédé selon la revendication 11, caractérisé en ce que l'alcool est un alcanol ayant 1 à 6 atomes de carbone.

13. Procédé selon la revendication 10, caractérisé en ce que l'alcool est choisi dans le groupe constitué par le méthanol, l'éthanol, le n- et le i-propanol, le n-, le i- et le t-butanol, le pentanol, l'hexanol, l'éther monométhylique et monoéthylique d'éthylène glycol ou de diéthylène glycol.

14. Procédé selon la revendication 11, caractérisé en ce que l'alcool est un alcanol ayant 1 à 4 atomes de carbone.

15. Procédé selon la revendication 14, caractérisé en ce que l'alcool est le méthanol.

16. Procédé selon la revendication 1, caractérisé en ce que l'alcool est un cycloalcanol ayant 5 à 12 atomes de carbone.

17. Procédé selon la revendication 16, caractérisé en ce que l'alcool est un cycloalcanol ayant 5 à 8 atomes de carbone.

18. Procédé selon la revendication 16, caractérisé en ce que l'alcool est choisi dans le groupe constitué par le cyclopentanol, le cyclohexanol, le méthylcyclohexanol, le cycloheptanol et le cyclooctanol.

19. Procédé selon la revendication 1, caractérisé en ce que l'alcool est un alcool araliphatique ayant 7 à 30 atomes de carbone.

20. Procédé selon la revendication 19, caractérisé en ce que l'alcool est un alcool araliphatique ayant 7 à 20 atomes de carbone.

21. Procédé selon la revendication 19, caractérisé en ce que l'alcool est choisi dans le groupe constitué par l'alcool benzylique non substitué, substitué par un alkyle ou un alcoxy, et le β-phényléthanol.

22. Procédé selon la revendication 1, caractérisé en ce que l'alcool est un alcool aromatique ayant 6 à 30 atomes de carbone.

23. Procédé selon la revendication 22, caractérisé en ce que l'alcool est un alcool aromatique ayant 6 à 12 atomes de carbone.

24. Procédé selon la revendication 22, caractérisé en ce que l'alcool est choisi dans le groupe constitué par le phénol, le naphtol, et le phénol ou le naphtol non substitué, substitué par un alkyle ou un alcoxy.

25. Procédé selon la revendication 1, caractérisé en ce que le polyol contient 2 à 20 atomes de carbone.

26. Procédé selon la revendication 25, caractérisé en ce que le polyol est choisi dans le groupe constitué par l'éthylène glycol, le propylène glycol, l'alcane diol, le pentane diol, l'hexane diol, le cyclohexane diol, le dihydrométhylcyclohexane, le diéthylène glycol, le triéthylène glycol, le polyéthylène glycol, le triméthylol propane, le pentaérythritol, le dipentaérythritol, la glycérine et la saccharine.

27. Procédé selon la revendication 1, caractérisé en ce que le polyol polymère est choisi dans le groupe constitué par l'alcool polyvinylique, les polysaccharides et les hydroxyalkyl esters d'acide polyacrylique ou selon les cas méthacrylique.

28. Procédé selon la revendication 1, caractérisé en ce que la base inorganique est choisie dans le groupe constitué par un oxyde métallique basique, un oxyde ou un hydroxyde de métal lourd.

29. Procédé selon la revendication 28, caractérisé en ce que la base inorganique est l'oxyde d'aluminium basique.

30. Procédé selon la revendication 28, caractérisé en ce que la base inorganique est l'oxyde d'aluminium basique.

31. Procédé selon la revendication 1, caractérisé en ce que la quantité catalytique de base se situe dans un intervalle de 0,01 à 20 % molaire.

32. Procédé selon la revendication 1, caractérisé en ce que la base azotée organique est choisie dans le groupe constitué par une amine aromatique, un N-hétérocycle aromatique et une N-alkyl morpholine.

33. Procédé selon la revendication 32, caractérisé en ce que la base est choisie dans le groupe constitué par l'imidazole, la pyridine, la polyvinylpyridine, la N-méthylmorpholine, l'aniline et le o-diaminobenzène.

34. Procédé selon la revendication 1, caractérisé en ce que les nucléosides et oligonucléosides sont liés à un support solide.

35. Procédé selon la revendication 1, caractérisé en ce qu'on trouve en outre des quantités catalytiques d'une base inorganique nucléophile, qui catalysent la réaction.

36. Procédé selon la revendication 1, caractérisé en ce qu'on trouve en outre des quantités catalytiques d'un acide de Lewis.
